(19) Europäisches Patentamt / European Patent Office / Office européen des brevets

(11) **EP 1 748 819 B1**

(12) **EUROPÄISCHE PATENTSCHRIFT**

(45) Veröffentlichungstag und Bekanntmachung des Hinweises auf die Patenterteilung:
**27.07.2016 Patentblatt 2016/30**

(21) Anmeldenummer: **05745186.6**

(22) Anmeldetag: **28.04.2005**

(51) Int Cl.:
**A61N 1/36** *(2006.01)*     **A61B 5/0476** *(2006.01)*

(86) Internationale Anmeldenummer:
**PCT/DE2005/000780**

(87) Internationale Veröffentlichungsnummer:
**WO 2005/115537 (08.12.2005 Gazette 2005/49)**

(54) **VORRICHTUNG ZUR ENTKOPPLUNG UND/ODER DESYNCHRONISATION NEURONALER HIRNAKTIVITÄT**

DEVICE FOR DECOUPLING AND/OR DESYNCHRONIZING NEURAL BRAIN ACTIVITY

DISPOSITIF POUR DECOUPLER ET/OU DESYNCHRONISER L'ACTIVITE CEREBRALE NEURONALE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **27.05.2004 DE 102004025945**

(43) Veröffentlichungstag der Anmeldung:
**07.02.2007 Patentblatt 2007/06**

(73) Patentinhaber: **Forschungszentrum Jülich GmbH**
**52425 Jülich (DE)**

(72) Erfinder:
- **TASS, Peter**
**81541 München (DE)**
- **POPOVYCH, Oleksandr**
**52355 Düren (DE)**
- **HAUPTMANN, Christian**
**52224 Stolberg (DE)**

- **KRACHKOVSKYI, Valerii**
**52428 Jülich (DE)**

(74) Vertreter: **Manitz, Finsterwald & Partner GbR**
**Martin-Greif-Strasse 1**
**80336 München (DE)**

(56) Entgegenhaltungen:
**WO-A-2005/053787     DE-A1- 10 211 765**
**DE-A1- 10 211 766     DE-A1- 10 233 960**
**DE-A1- 10 318 071**

- **TASS P A: "Desynchronizing double-pulse phase resetting and application to deep brain stimulation" BIOLOGICAL CYBERNETICS, SPRINGER VERLAG, BERLIN, DE, Bd. 85, Nr. 5, November 2001 (2001-11), Seiten 343-354, XP002245895 ISSN: 0340-1200**

**Beschreibung**

Die Erfindung betrifft eine Vorrichtung zur Entkopplung und/oder Desynchronisation von neuronaler Hirnaktivität nach dem Oberbegriff des Anspruchs 1.

Eine pathologisch synchrone Hirnaktivität, wie sie beispielsweise in den Basalganglien ihren Ursprung haben kann, kann als treibende Kraft eine Synchronisation auch in nachgeschalteten Arealen, wie beispielsweise dem Motorkortex bewirken. Diese sekundäre Synchronisation ist maßgeblich an der Erzeugung der pathologischen Symptome beteiligt. Die Erfindung betrifft eine Vorrichtung, die es erlaubt, die treibende, pathologische Aktivität von den nachgeschalteten Arealen zu entkoppeln, wodurch eine starke Verringerung der pathologischen Symptome bewirkt werden kann. In einer weiteren Ausführungsform kann die erfindungsgemäße Vorrichtung auch für die Desynchronisation, d. h. für die Unterdrückung einer rhythmischen, kollektiven Aktivität bzw. eines kollektiven Feuerns der Neuronen der pathologisch synchronen Nervenzellpopulationen, die als treibende Populationen bezeichnet werden, verwendet werden.

Bei Patienten mit neurologischen oder psychiatrischen Erkrankungen, z. B. Morbus Parkinson, essentiellem Tremor, Dystonie oder Zwangserkrankungen, sind Nervenzellverbände in umschriebenen Bereichen des Gehirns, z. B. des Thalamus und der Basalganglien krankhaft aktiv, z. B. übersteigert synchron. In diesem Fall bildet eine große Anzahl von Neuronen synchrone Aktionspotentiale aus; die beteiligten Neuronen feuern übermäßig synchron. Beim Gesunden hingegen feuern die Neuronen in diesen Hirngebieten qualitativ anders, z. B. auf unkorrelierte Weise. Die pathologisch synchrone Hirnaktivität verändert die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien den Großhirnarealen ihren Rhythmus auf, so dass schließlich die von diesen Arealen gesteuerten Muskeln pathologische Aktivität, z. B. ein rhythmisches Zittern (Tremor), entfalten.

Bei Patienten, welche medikamentös nicht (mehr) behandelt werden können, wird, je nach Krankheitsbild und je nach dem, ob die Erkrankung einseitig oder beidseitig auftritt, eine Tiefenelektrode einseitig oder beidseitig implantiert. Unter der Haut führt dabei ein Kabel vom Kopf zum sogenannten Generator, welcher ein Steuergerät mit einer Batterie umfasst, und beispielsweise im Bereich des Schlüsselbeins unter der Haut implantiert ist. Über die Tiefenelektroden wird eine Dauerreizung mit einer hochfrequenten periodischen Abfolge (pulse train mit einer Frequenz von > 100 Hz) von Einzelpulsen, z. B. Rechteckpulsen, durchgeführt. Ziel dieser Methode ist es, das Feuern der Neuronen in den Zielgebieten zu unterdrücken. Der Wirkmechanismus, welcher der Standard-Tiefenstimulation zugrunde liegt, ist noch nicht hinreichend geklärt. Die Ergebnisse mehrerer Studien sprechen dafür, dass die Standard-Tiefenstimulation wie eine reversible Läsionierung, d. h. wie eine reversible Ausschaltung des Gewebes, wirkt: Die Standard-Tiefenstimulation unterdrückt das Feuern der Neuronen in den Zielgebieten und/oder in damit verbundenen Hirnarealen.

Nachteilig bei dieser Stimulationsform ist, dass der Energieverbrauch des Generators sehr hoch ist, so dass der Generator inklusive Batterie häufig schon nach ca. ein bis drei Jahren operativ ausgetauscht werden muss. Noch nachteiliger ist, dass die Hochfrequenz-Dauerstimulation als unphysiologischer (unnatürlicher) Input im Bereich des Gehirns, z. B. des Thalamus bzw. der Basalganglien, im Laufe von wenigen Jahren zur Adaptation der betroffenen Nervenzellverbände führen kann. Um denselben Stimulationserfolg zu erzielen, muss dann infolge dieser Adaptation mit höherer Reizamplitude stimuliert werden. Je größer die Reizamplitude ist, desto größer ist die Wahrscheinlichkeit, dass es infolge der Reizung von Nachbararealen zu Nebenwirkungen - wie Dysarthrie (Sprechstörungen), Dysästhesie (zum Teil sehr schmerzhafte Missempfindungen), zerebelläre Ataxie (Unfähigkeit, ohne fremde Hilfe sicher zu stehen) oder Schizophrenie artigen Symptomen etc. - kommt. Diese Nebenwirkungen können vom Patienten nicht toleriert werden. Die Behandlung verliert daher in diesen Fällen nach wenigen Jahren ihre Wirksamkeit.

Bei anderen Stimulationsmethoden, wie sie beispielsweise in DE 102 11 766 A1 beschrieben sind, wurde vorgeschlagen, dass bedarfsgesteuert Reize im jeweiligen Zielgebiet appliziert werden. Das Ziel dieser Verfahren und dieser Vorrichtungen ist es, das krankhaft synchrone Feuern nicht- wie bei der Standard-Tiefenstimulation - einfach zu unterdrücken, sondern näher an das physiologische, unkorrelierte Feuermuster heran zu bringen. Hierdurch soll einerseits der Stromverbrauch vermindert werden und andererseits durch die bedarfsgesteuerte Stimulation der Energieeintrag in das Gewebe im Vergleich zur Standard-Tiefenstimulation reduziert werden.

Bei den oben erwähnten Stimulationsmethoden ist die Verwendung von einer oder mehreren Tiefenelektroden notwendig, was einen hohen operativen Aufwand und ein großes Risiko von Komplikationen wie, z.B. mögliche Hirngewebsschädigungen oder eine Hirnblutung während der Implantation der Tiefelektroden, für den Patienten darstellt. Dieses Risiko sollte im Sinne einer erfolgreichen Heilung des Patienten und einer Verminderung von Nebenwirkungen aber vermindert werden.

Aufgabe der Erfindung ist es daher, eine Vorrichtung zur Entkopplung und/oder Desynchronisation von neuronaler Hirnaktivität zu schaffen, mit der Patienten mit krankhaft synchronisierter Hirnaktivität mild und effizient behandelt werden können. Hierbei soll eine Adaptation an einen unphysiologischen Dauerreiz unterbunden werden. Es sollen langwierige Kalibrierungsvorgänge verhindert werden und die Stimulation soll auch dann erfolgreich sein, wenn die Hauptfrequenz-Komponente der pathologisch rhythmischen Aktivität starken Schwankungen unterliegt. Des Weiteren soll die Vorrichtung eine dauerhafte Entkopplung und/oder Desynchronisation erreichen, transiente, stimulationsbedingte unphysiologische Zustände sollen weitestgehend vermieden werden. Die erfindungsgemäße Vorrichtung bedarf keiner zusätzlichen Bedarfssteuerung, die wie in Abschnitt 6.3 beschrieben, optional hinzugefügt werden kann, daher ist sie technisch leicht realisierbar und es werden nur geringe Ansprüche an die Komplexität der Steuerelektronik und damit

auch an den Stromverbrauch gestellt. Die erfindungsgemäße Stimulationsvorrichtung soll Strom sparend funktionieren, so dass die Batterien des in den Patienten implantierten Stimulators seltener operativ ausgetauscht werden müssen. Da eine Implantation nur einer Elektrode notwenig ist, und da diese Elektrode in einem nachgeschalteten und somit möglicherweise leichter zugänglichen Hirnareal implantiert wird, wie, z.B. eine Epikortikalelektrode im Bereich des Motor Cortex, stellt die erfindungsgemäße Vorrichtung eine erhebliche Verbesserung im Vergleich zu den oben erwähnten Methoden der Tiefenhirnstimulation dar. Für die Hirnstimulation ist nämlich insbesondere - in einer besonderen Ausführungsform der erfindungsgemäßen Vorrichtung - keine Tiefenelektrode notwendig, so dass keine intraoperative Blutungsgefahr durch eine Verletzung einer Arterie gegeben ist.

Ausgehend vom Oberbegriff des Anspruchs 1 wird die Aufgabe erfindungsgemäß gelöst durch die im kennzeichnenden Teil des Anspruchs 1 angegebenen Merkmale. Unter Verwendung der gemessenen und bearbeiteten Aktivität der zu entkoppelnden und/oder der zu desynchronisierenden Neuronenpopulation als Feedback-Stimulationssignal, siehe Abschnitt 3, wird die Aufgabe überraschenderweise dadurch gelöst, dass die Neuronen mit einer Elektrode durch die Stimulation mit dem Feedback-Stimulationssignal in ihrer Aktivität jeweils derart beeinflusst werden, dass es überraschenderweise zu einer vollständigen Entkopplung und/oder Desynchronisation der zu entkoppelnden Neuronenpopulation von der treibenden, pathologischen Neuronenpopulation kommt, wodurch bei einem Patienten überraschenderweise die Symptomatik unterdrückt wird. Bei einer weiteren Ausführungsform der erfindungsgemäßen Vorrichtung, wie in Abschnitt 8 beschrieben, kann die Vorrichtung beispielsweise auch zur Desynchronisation der treibenden Neuronenpopulation verwendet werden. Bei dieser Ausführungsform wird die gemessene und bearbeitete neuronale Aktivität der treibenden Neuronenpopulation als Feedback-Stimulationssignal über die Stimulationselektrode appliziert, so dass eine direkte oder indirekte Stimulation der treibenden Neuronenpopulation mit dem Feedback-Stimulationssignal erfolgt. Dadurch wird die zu desynchronisierende Neuronenpopulation so beeinflusst, dass es überraschenderweise zu einer vollständigen Desynchronisation kommt, wodurch die krankheitsbedingte Symptomatik unterdrückt wird. Hierzu umfasst die erfindungsgemäße Vorrichtung eine Steuerung 4, die das Messsignal des Sensors 3 oder der Sensoren 3 aufnimmt und aus diesem Signal ein Stimulationssignal generiert und an die Elektrode 2 als Stimulationsreiz weiter gibt.

Die erfindungsgemäße Vorrichtung arbeitet Strom sparend, so dass im Patienten implantierte Batterien seltener ausgetauscht werden müssen.

Die erfindungsgemäße Vorrichtung ermöglicht es, den mit der entkoppelnden Stimulation intraoperativ erzielten Effekt zur Auswahl des am besten geeigneten Zielpunkts für die Elektrode zu verwenden. Bei Verwendung einer Tiefenhirnelektrode als Elektrode 2 wird während der Implantation der Elektrode im Bereich des anatomisch vorberechneten Zielpunkts in mm-Schritten vorangehend eine Testreizung und/oder Ableitung des Feedback-Signals mit der erfindungsgemäßen Vorrichtung durchgeführt. Der Zielpunkt, bei welchem sich der beste therapeutische Effekt erzielen lässt, wird als Zielpunkt für die dauerhafte Implantation gewählt.

Neben den oben genannten Erkrankungen, die häufig anhaltende pathologisch synchrone Aktivität mit relativ konstanter Frequenz aufweisen, können auch Erkrankungen behandelt werden, bei denen es nur intermittent (kurzzeitig auftretend) zu pathologisch synchroner Aktivität kommt. Eine Hauptindikation ist dabei die Behandlung von medikamentös nicht (mehr) behandelbaren Epilepsien. Die erfindungsgemäße Vorrichtung kann beispielsweise bei den Krankheiten Morbus Parkinson, essentieller Tremor, Dystonie, Epilepsie, Depression und Zwangserkrankungen eine Unterdrückung der Symptome bewirken.

Vorteilhafte Weiterbildungen der Erfindung sind in den Unteransprüchen angegeben.

Die Figuren zeigen beispielhafte Ausführungsformen der Erfindung. Es zeigt:

Fig.1: Eine erfindungsgemäße Vorrichtung

Figur 2 zeigt die entkoppelnde Wirkung einer Stimulation mit einem Stimulationsreiz wie er im Beispiel 1 in Abschnitt 8.1 beschrieben wird. Zur Veranschaulichung wird in Figur 2a bis 2d die Kopplung zum Zeitpunkt 4 Sekunden eingeschaltet, die Stimulation beginnt zum Zeitpunkt 7.5 Sekunden.

Fig.2a: Zeitgang der über Sensor 3 gemessenen neuronalen Aktivität der zu entkoppelnden Neuronenpopulation während des nicht gekoppelten Zustands, während der Kopplung und während der Stimulation.

Fig.2b: Zeitgang des Feuermusters der zu entkoppelnden Neuronenpopulation während des nicht gekoppelten Zustands, während der Kopplung und während der Stimulation.

Fig.2c: Zeitgang des Synchronisationsmaßes der zu entkoppelnden Neuronenpopulation während eines Stimulationsintervalls. Kleine Werte entsprechen geringer Synchronisation und große Werte entsprechen einer starken Synchronisation.

Fig.2d: Zeitgang des resultierenden Stimulationseinflusses auf die zu entkoppelnde Neuronenpopulation, d.h. die Summe der Kopplungs- und Stimulationseinflüsse.

Fig.2e: Verteilung der Feuerfrequenzen vor der Kopplung (links), während der Kopplung (Mitte) und bei eingeschalteter Stimulation (rechts).

Figur 3 zeigt die entkoppelnde Wirkung einer Stimulation mit einem Stimulationsreiz wie er im Beispiel 2 in Abschnitt 8.1 beschrieben wird. Zur Veranschaulichung wird in Figur 3a bis 3d die Kopplung zum Zeitpunkt 4 Sekunden eingeschaltet, die Stimulation beginnt zum Zeitpunkt 7.5 Sekunden.

Fig.3a: Zeitgang der über Sensor 3 gemessenen neuronalen Aktivität der zu entkoppelnden Neuronenpopulation während des nicht gekoppelten Zustands, während der Kopplung und während der Stimulation.

Fig.3b: Zeitgang des Feuermusters der zu entkoppelnden Neuronenpopulation während des nicht gekoppelten Zustands, während der Kopplung und während der Stimulation.

Fig.3c: Zeitgang des Synchronisationsmaßes der zu entkoppelnden Neuronenpopulation. Kleine Werte entsprechen geringer Synchronisation und große Werte einer starken Synchronisation.

Fig.3d: Zeitgang des resultierenden Stimulationseinflusses auf die zu entkoppelnde Neuronenpopulation, d.h. die Summe der Kopplungs- und Stimulationseinflüsse.

Fig.3e: Verteilung der Feuerfrequenzen vor der Kopplung (links), während der Kopplung (Mitte) und bei eingeschalteter Stimulation (rechts).

Fig.4: Schematische Zeichnung der Kopplung zwischen der treibenden, pathologisch synchronen Neuronenpopulation 1 und der getriebenen, zu entkoppelnden Neuronenpopulation 2. Beispielsweise stellt die Neuronenpopulation 2 den prämotorischen Kortex und/oder den Motorkortex dar.

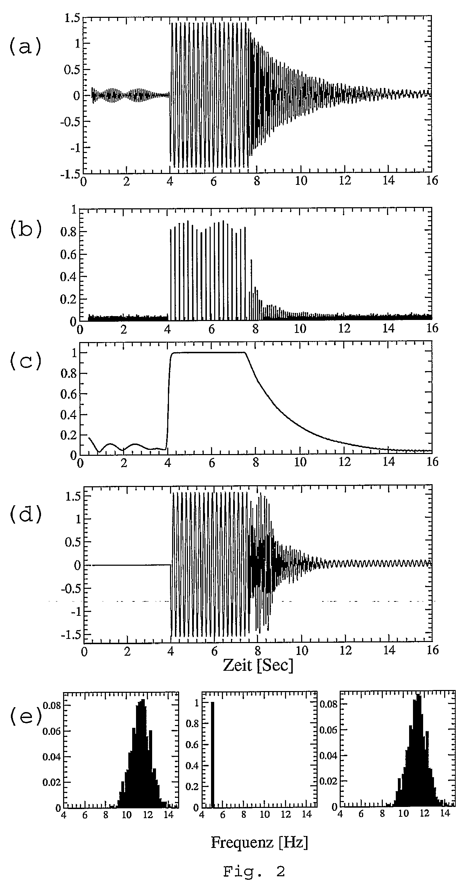

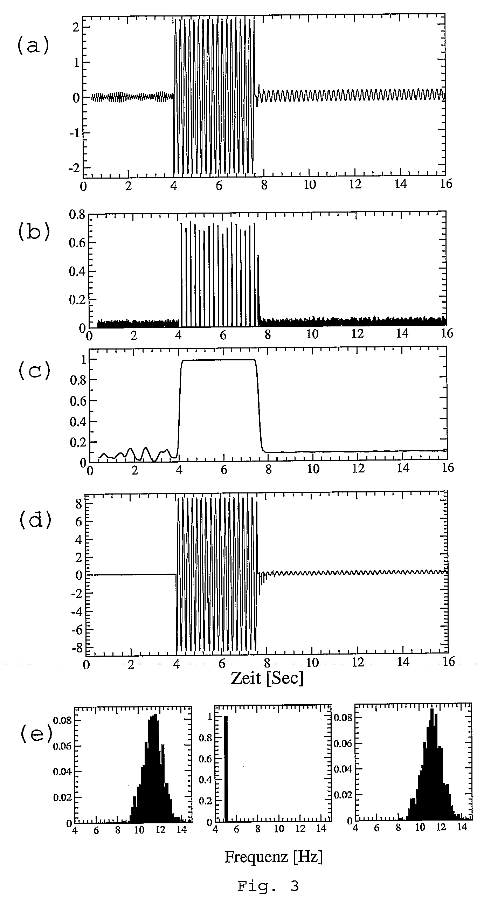

In den Figuren 2a bis d und 3a bis d bezeichnen die Abszissen die Zeitachsen in Sekunden, während auf den Ordinaten die gemessene neuronale Aktivität (Fig.2a, 3a) bzw. das Feuermuster (Fig.2b, 3b) bzw. das Synchronisationsmaß (Fig.2c, 3c) bzw. die Summe der Kopplungs- und Stimulationseinflüsse (Fig.2d, 3d) jeweils in willkürlichen Einheiten aufgetragen sind. Die über Sensor 3 gemessene neuronale Aktivität (Fig.2a, 3a) dient als Grundlage zur Erstellung des Stimulationsreizes. In den Figuren 2e und 3e bezeichnen die Abszissen die Frequenz und die Ordinaten bezeichnen die relative Zahl der Neuronen mit entsprechender Frequenz.

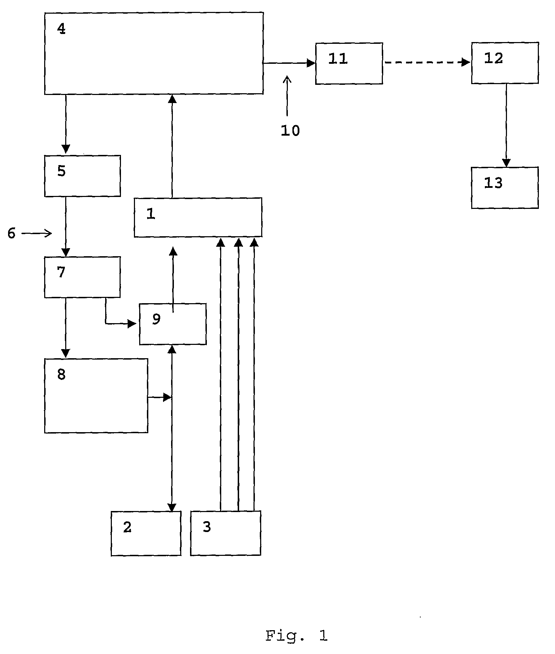

Die Vorrichtung gemäß Figur 1 umfasst einen Trennverstärker 1, an dem eine Elektrode 2 sowie mindestens einen Sensor 3 zur Erfassung von physiologischen Messsignalen angeschlossen sind. Als Elektrode 2 kann beispielsweise eine epikortikale Elektrode oder Hirnelektrode eingesetzt werden. Der Trennverstärker steht weiterhin mit einer Einheit 4 zur Signalverarbeitung und Steuerung in Verbindung, welche an einen optischen Sender für die Stimulation 5 angeschlossen ist. Der optische Sender 5 ist über Lichtwellenleiter 6 mit einem optischen Empfänger 7 verbunden, welcher mit einer Stimulatoreinheit 8 zur Signalerzeugung in Verbindung steht. Die Stimulatoreinheit 8 für die Signalerzeugung steht mit einer Elektrode 2 in Verbindung. Am Eingangsbereich der Elektrode 2 in den Trennverstärker 1 befindet sich ein Relais 9 oder Transistor. Die Einheit 4 steht über eine Leitung 10 mit einem Telemetriesender 11 in Verbindung, welcher mit einem Telemetrieempfänger 12 in Verbindung steht, der sich außerhalb des zu implantierenden Geräts befindet und an den ein Mittel zur Visualisierung, Verarbeitung und Speicherung der Daten 13 angeschlossen ist. Als Sensoren 3 können beispielsweise epikortikale Elektroden, Hirnelektroden oder periphere Elektroden eingesetzt werden.

Bei der Elektrode 2 kann es sich um jede Elektrode handeln, die dem Fachmann bekannt ist und die für die erfindungsgemäße Anwendung geeignet ist. Im weiteren Sinne der Erfindung ist eine Elektrode daher ein Gegenstand, der die erfindungsgemäßen Reize applizieren kann.

Bei der Elektrode 2 handelt es sich beispielsweise um mindestens zwei Drähte, an deren Enden eine Potentialdifferenz zum Zwecke der Stimulation angelegt wird. Es kann sich dabei um eine Makro- oder Mikroelektrode handeln. Alternativ kann es sich bei der Elektrode 2 auch um einen einzelnen Draht handeln. In diesem Fall wird zum Zwecke der Stimulation eine Potentialdifferenz zwischen einem einzelnen Draht und dem metallischen Teil des Gehäuses des Generators angelegt. Zusätzlich, aber nicht zwingend, kann über die Elektrode 2 eine Potentialdifferenz gemessen werden, um eine neuronale Aktivität zu registrieren. In einer weiteren Ausführungsform können die Elektrode 2 auch aus mehr als zwei einzelnen Drähten bestehen, die sowohl für die Ermittlung eines Messsignals im Gehirn, als auch für die Stimulation herangezogen werden können.

Für den Fall, dass die Elektrode 2 mehr als zwei Drähte umfassen, können mindestens einer dieser Drähte auch als Sensor 3 fungieren, so dass in diesem Fall eine Ausführungsform vorliegt, bei der die Elektrode 2 und der Sensor 3 in einem einzigen Bauteil vereint sind. Die Drähte der Elektrode 2 können unterschiedliche Längen haben, so dass sie in verschiedene Hirntiefen eindringen können. Besteht die Elektrode 2 aus n Drähten, wobei n eine ganze Zahl größer als 2 ist, so kann eine Stimulation über mindestens ein Paar von Drähten erfolgen, wobei bei der Paarbildung jede Unterkombination von Drähten möglich ist. Es kann auch eine Stimulation zwischen einem der n Drähte und dem metallischen Teil des Gehäuses des Generators durchgeführt werden. Neben diesem Bauteil können auch zusätzlich nicht mit der Elektrode 2 baulich vereinte Sensoren 3 vorhanden sein.

Beispielhaft und anschaulich gesprochen wird durch die erfindungsgemäße Vorrichtung in einem ersten Schritt mittels der Sensoren die neuronale Aktivität gemessen. In einem zweiten Schritt wird das Stimulationssignal durch weitere Bearbeitung des gemessenen Signals, z.B. durch zeitliche Verzögerung und gegebenenfalls durch Filterung und/oder Verstärkung der neuronalen Aktivität, generiert. Über eine implantierte Elektrode wird der aus diesem Stimulationssignal generierte Stimulationsreiz sodann in einem dritten Arbeitsschritt zur Stimulation verwendet. Als Folge dieser Stimulation tritt im stimulierten Gewebe eine Entkopplung und/oder Desynchronisation der pathologischen Aktivität ein. Details der Funktionsweise der erfindungsgemäßen Vorrichtung sind in Abschnitt 1 erläutert.

Wie in Abschnitt 6 beschrieben, kann die erfindungsgemäße Vorrichtung in verschiedenen Ausführungsformen der zeitlichen Steuerung der Reizapplikation realisiert werden. Die Varianten der zeitlichen Steuerung der Reizapplikation sind permanente, wiederkehrende und bedarfsgesteuerte Reizapplikation.

Die erfindungsgemäße permanente Reizapplikation ist eine einfache Ausführungsform der erfindungsgemäßen Vorrichtung, die ohne zusätzliche Bedarfssteuerung arbeitet und permanent, wie in Abschnitt 6.1 beschrieben, Reize appliziert. Somit stellt die permanente Reizapplikation eine leicht zu realisierende Ausführungsform der erfindungsgemäßen Vorrichtung dar. Gleichzeitig erfolgt aufgrund der in Abschnitt 5 beschriebenen erfindungsgemäßen selbstregulierenden Bedarfssteuerung eine gute entkoppelnde und/oder desynchronisierende Wirkung der permanenten Stimulation bei geringem Energieeintrag in die zu entkoppelnde oder in die zu desynchronisierende Population.

Bei der erfindungsgemäßen wiederkehrenden Reizapplikation verfügt die erfindungsgemäße Vorrichtung über eine Steuerung, die so programmiert ist, dass sie an der Elektrode 2 eine Applikation des Stimulationsreizes nur während bestimmter Zeitintervalle vornimmt. Außerhalb dieser Zeitintervalle erfolgt keine Stimulation. Die Steuereinheit 4 ist daher so programmiert, dass in der Ausführungsform der in Abschnitt 6.2 beschriebenen wiederkehrenden Stimulation zu von Steuereinheit 4 bestimmten, beispielsweise periodisch aufeinander folgenden Zeitpunkten ein Stimulationssignal mit einer von Steuereinheit 4 berechneten Dauer generiert und an die Elektrode 2 abgegeben wird. Wie im Fall der permanenten Reizapplikation findet die selbstregulierende Bedarfssteuerung des Stimulationssignals gemäß Abschnitt 5 auch bei der wiederkehrenden Reizapplikation statt.

Bei der erfindungsgemäßen bedarfsgesteuerten Reizapplikation verfügt die erfindungsgemäße Vorrichtung über eine zusätzliche Bedarfssteuerung wie im Abschnitt 6.3 beschrieben. Dafür ist die erfindungsgemäße Vorrichtung vorzugsweise mit Mitteln zum Erkennen des Auftretens und/oder der Ausprägung der pathologischen Merkmale in den Signalen der Elektrode 2 und/oder in der Sensoren 3 und/oder in der bearbeiteten neuronalen Aktivität ausgestattet. In Abhängigkeit vom Auftreten und der Ausprägung pathologischer Merkmale wird in der Ausführungsform der in Abschnitt 6.3 beschriebenen bedarfsgesteuerten Reizapplikation ein Reizsignal an die Elektrode 2 abgegeben, so dass eine Stimulation des Hirngewebes erfolgt. Dadurch wird die pathologische neuronale Aktivität in der Neuronenpopulationen entkoppelt und/oder desynchronisiert und somit dem natürlichen, physiologischen Zustand näher gebracht. Die pathologische Aktivität unterscheidet sich von der gesunden Aktivität durch eine charakteristische Veränderung ihres Musters und/oder ihrer Amplitude und/oder ihres Frequenzgehalts und/oder im ihrem Zeitgang. Die Mittel zum Erkennen des pathologischen Musters sind dabei ein Rechner, der die gemessenen Signale der Elektrode 2 und/oder des Sensors 3 verarbeitet und mit im Rechner gespeicherten Daten vergleicht. Der Rechner verfügt über einen Datenträger, welcher Daten speichert. Diese können im Rahmen der Kalibrierung und/oder Steuerung gemäß der Abschnitte 6 und 7 benutzt werden. Die Steuereinheit 4 kann beispielsweise einen Chip oder eine andere elektronische Vorrichtung mit vergleichbarer Rechenleistung umfassen.

Die Steuereinheit 4 ist so programmiert, dass in der Ausführungsform der in Abschnitt 6.3 beschriebenen bedarfsgesteuerten Reizapplikation in einem von Steuereinheit 4 vorgegebenen Stimulationsintervall einen Stimulationsreiz generiert und an die Elektrode 2 abgegeben wird. Insgesamt sollen alle für die jeweilige Verfahrensweise der erfindungsgemäßen Vorrichtung relevanten Parameter für die Art und Stärke der Stimuli, als auch deren zeitliche Verzögerungen sowie Information zur elektrodenspezifischen Applikation als auch die für die bedarfsgesteuerte Funktionsweisen relevanten, über Sensor 3 ermittelten Messwerte bzw. daraus abgeleiteten Parameter abgespeichert werden.

Die Steuereinheit 4 steuert die Elektrode 2 vorzugsweise in folgender Weise an: Die Steuerdaten werden von der Steuereinheit 4 an einen optischen Sender für die Stimulation 5 weitergegeben, welcher über den Lichtleiter 6 den optischen Empfänger 7 ansteuert. Durch das optische Einkoppeln von Steuersignalen in den optischen Empfänger 7, wird eine galvanische Trennung der Steuereinheit 4 von den Elektrode 2 bewirkt. Dies bedeutet, dass eine Einstreuung von Störsignalen von der Einheit zur Signalverarbeitung und Steuerung 4 in die Elektrode 2 verhindert wird. Als optischer Empfänger 7 kommt beispielsweise eine Photozelle in Betracht. Der optische Empfänger 7 gibt die über den optischen Sender für die Stimulation 5 eingegebenen Signale an die Stimulatoreinheit 8 weiter. Über die Stimulatoreinheit 8 werden dann gezielte Stimuli über die Elektrode 2 an das Zielareal im Gehirn weitergegeben. Für den Fall, dass über die Elektrode 2 auch gemessen wird, wird ausgehend vom optischen Sender für die Stimulation 5 über den optischen Empfänger 7 auch ein Relais 9 angesteuert, wodurch die Einstreuung von Störsignalen verhindert wird. Das Relais 9 oder der Transistor stellt sicher, dass die neuronale Aktivität unmittelbar nach jedem Stimulus wieder gemessen werden kann, ohne dass der Trennverstärker übersteuert. Die galvanische Trennung muss nicht zwingend durch eine optische Einkopplung der Steuersignale erfolgen, vielmehr können auch andere alternative Steuerungen verwendet werden. Diese können beispielsweise akustische Verbindungen zum Beispiel im Ultraschallbereich sein. Eine störungsfreie Steuerung kann auch beispielsweise unter Zuhilfenahme geeigneter analoger oder digitaler Filter realisiert werden.

Weiterhin steht die erfindungsgemäße Vorrichtung vorzugsweise mit Mitteln zur Visualisierung und Verarbeitung der Mess- und/oder Stimulationssignale sowie zur Datensicherung 13 über den Telemetrieempfänger 12 in Verbindung. Dabei kann die Einheit 13 über die unten erwähnten Verfahren zur Datenanalyse verfügen.

Weiterhin kann die erfindungsgemäße Vorrichtung über den Telemetrieempfänger 13 mit einer zusätzlichen Referenzdatenbank in Verbindung stehen, um beispielsweise den ordnungsgemäßen Betrieb des Gerätes zu überwachen und ggf. die in Abschnitt 7.2 beschriebenen Steuermechanismen durch Modifikation der Parameter effizienter auszugestalten.

In Abschnitt 1 wird der Mechanismus der Stimulation ausführlich erläutert. Definitionen der wichtigsten Begriffe sind in Abschnitt 2 zu finden. Die Arbeitsschritte von der Messung der neuronalen Aktivität über deren Bearbeitung bis zu der Generierung des Stimulationssignals ist in Abschnitt 3 erläutert. Die räumliche Anordnung der Elektrode und Sensoren ist Gegenstand von Abschnitt 4. Abschnitt 5 befasst sich mit der selbstregulierenden Bedarfssteuerung der Stimulationssignale. Die Steuerung der Reizapplikation und die Kalibrierung und Anpassung der Stimulationsparameter wird in den Abschnitten 6 und 7 beschrieben. In Abschnitt 8 werden Beispiele und weitere Verwendungsmöglichkeiten und Ausführungsformen der Vorrichtung erläutert. Die Vorteile der erfindungsgemäßen Vorrichtung sind in Abschnitt 9 angeführt.

## 1 Mechanismus der Stimulation

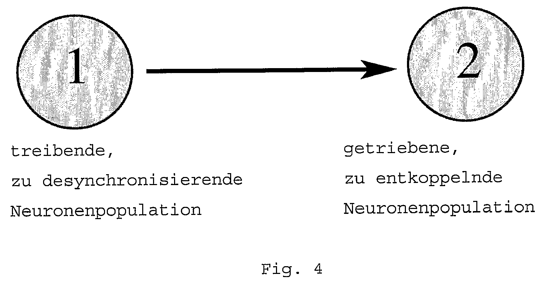

Mit dem erfindungsgemäßen Verfahren und der Vorrichtung kann die getriebene Neuronenpopulation von der treibenden Neuronenpopulation entkoppelt werden. Es kann auch die treibende Neuronenpolulation desynchronisiert werden. Diese Beziehung ist in Figur 4 dargestellt.

Dies geschieht, in dem mittels einer Elektrode Reize appliziert werden, die dadurch generiert werden, dass neuronale Aktivität gemessen und nach gegebenenfalls vorhandenen Bearbeitungsschritten, die vorzugsweise auch eine zeitliche Verzögerung einschließen, in ein Stimulationssignal und weiter in einen Stimulationsreiz umgewandelt und appliziert wird, so dass sich überraschenderweise eine Entkopplung und/oder Desynchronisation einstellt. Wie in Abschnitt 3.1 beschrieben, wird bei entkoppelnden Verfahrensweise die getriebene Nervenzellpopulation 2 (Fig.4) stimuliert. Bei der desynchronisierenden Verfahrensweise wird die treibende Neuronenpopulation 1 stimuliert. Mit der erfindungsgemäßen Vorrichtung und dem erfindungsgemäßen Stimulationsverfahren wird die zu entkoppelnde Nervenzellpopulation unmittelbar in einen entkoppelten und desynchronisierten Zustand gebracht oder die zu desynchronisierende Population wird desynchronisiert. Der gewünschte Zustand, das heißt die komplette Entkopplung und/oder Desynchronisation, stellt sich typischerweise, während weniger Perioden der neuronalen Aktivität, häufig in weniger als einer Periode ein. Typischerweise besteht die Notwendigkeit permanenter oder wiederkehrender Stimulation, da die zu entkoppelnde und/oder die zu desynchronisierende Nervenzellpopulation nach Ausschalten der Stimulation erfahrungsgemäß krankheitsbedingt und/oder aufgrund der Kopplung wieder resynchronisiert. Da die Stimulation erfindungsgemäß direkt mit der neuronalen Aktivität zusammenhängt, wird die Amplitude des resultierenden Stimulationseinflusses, d.h. die Summe der Kopplung und Stimulation, auf die zu entkoppelnde oder auf die zu desynchronisierende Neuronenpopulation nach erfolgreicher Entkopplung und/oder Desynchronisation automatisch minimiert. Dies wird ermöglicht, dadurch dass als Stimulationsreiz das Feedback-Stimulationssignal, das heißt die bearbeitete neuronale Aktivität, benutzt wird, d.h. das Ausmaß der Synchronisation und damit der Kopplung steuert permanent die Stärke und Form des Stimulationssignals. Das applizierte Stimulationssignal kompensiert die Kraft der externen Kopplung und/oder der internen Synchronisation, so dass die Amplitude des resultierenden Stimulationseinflusses auf die zu entkoppelnde oder auf die zu desynchronisierende Neuronenpopulation minimiert wird und deren neuronale Aktivität näher zum natürlichen, physiologischen Zustand kommt. Dieser Prozess funktioniert für einen großen Bereich der modifizierbaren Stimulationsparameter, wie beispielsweise Stimulationsperiode T, die Zeitverzögerung und die Intensität, benötigt keine aufwendige Kalibrierung und verfügt über eine große Fehlertoleranz. Des Weiteren wird der Energieeintrag in das zu entkoppelnde oder in das zu desynchronisierende Gewebe aufgrund des direkten Zusammenhangs zwischen neuronaler Aktivität und Stimulationsmuster minimiert, was geringere Nebenwirkungen erwarten lässt.

Im Folgenden soll die erfindungsgemäße Vorrichtung und deren Funktionieren beispielhaft erläutert werden.

Die erfindungsgemäße Vorrichtung und die Steuerung sind mit Mitteln ausgestattet, die alle Schritte des erfindungsgemäßen Behandlungsverfahrens durchführen können. Mit den offenbarten Verfahrensschritten sollen daher implizit auch Mittel zur Durchführung des Verfahrensschrittes offenbar sein. Die Verfahrensschritte stellen somit auch gleichzeitig die funktionalisierten Vorrichtungsmerkmale dar.

Erfindungsgemäß wird eine Elektrode in ein Hirnareal eingebracht oder - im Fall einer epikortikale Elektrode - auf einem Hirnareal befestigt. Vorzugsweise wird dieses Hirnareal so gewählt, dass es direkt oder indirekt mit einer oder mehrere Hirnregionen verbunden ist oder unmittelbar zu einer dieser Regionen gehört, welche für die Ausbildung des Krankheitsbildes verantwortlich sind oder von der pathologischen Aktivität getrieben sind.

Die Elektrode gibt dabei in ihrer Umgebung ein elektrisches Signal ab, welches entweder direkt in ihrem Umfeld oder über ein Nervenfaserbündel fortgeleitet in einem anderen Areal eine Entkopplung und/oder eine Desynchronisation bewirkt. Um eine Entkopplung und/oder Desynchronisation herbeizuführen, wird die gemessene und bearbeitete, vorzugsweise zeitlich verzögerte, neuronale Aktivität, siehe Abschnitt 3, als Stimulationssignal verwendet. Die erfindungsgemäße Vorrichtung verfügt daher über eine Steuerung, welche die Elektrode 2 so ansteuert, dass sie in ihrem näheren Umfeld und/oder durch Weiterleitung der Stimulation über ein Faserbündel in einem anderen Gehirnareal eine Entkopplung und/oder eine Desynchronisation bewirken. Erfindungsgemäß wird die Elektrode mit Stimulationsreizen angesteuert, die aus der gemessenen und bearbeiteten neuronalen Aktivität mit vorzugsweise einer Zeitverzögerung von einem ganzzahligen Vielfachen von $T/2$ gebildet werden. $T$ ist hierbei die Stimulationsperiode und approximiert, wie unten beschrieben, im Wesentlichen die Periode der rhythmischen neuronalen Aktivität der treibenden oder getriebenen Neuronenpopulation. Falls sich die stimulierende Elektrode 2 nicht in dem zu entkoppelnden und in dem zu desynchronisierenden Areal befindet, ist bei der Ansteuerung einer solchen Elektrode 2 die Laufzeit zwischen dem Reizort, und dem Ort der hierdurch beeinflussten Neuronenpopulation zu berücksichtigen. Dies wird in Abschnitt 7.3 beschrieben. Überraschenderweise kommt es bei dieser Stimulation zu einer Entkopplung und Desynchronisation der gesamten, zu entkoppelnden Neuronenpopulation und/oder zu einer Desynchronisation der zu desynchronisierenden Neuronenpopulation, was mit einer Unterdrückung der pathologischen Symptome einhergeht. Befindet sich die Elektrode 2 außerhalb des zu entkoppelnden und des zu desynchronisierenden Areals, so müssen Effekte der indirekten Stimulation, wie sie in Abschnitt 7.3 beschrieben sind, berücksichtigt werden.

Mit dem neuen Verfahren und der neuen Vorrichtung wird im Vergleich zum oben genannten Stand der Technik auf eine qualitativ andere Art das Ziel, die pathologischen Symptome zu unterdrücken, erreicht. Anstatt die neuronale Aktivität des krankhaft synchronen Nervenzellverbands mit einem starken Stimulationsreiz zu unterdrücken, wird der krankhaft synchrone, treibende Nervenzellverband einfach desynchronisiert oder es wird eine andere von der pathologischen Aktivität getriebene Neuronenpopulation wird von dieser Kraft entkoppelt und desynchronisiert, was zu einer Unterdrückung der pathologischen Symptome führt. Die physiologischen Aktivitäten der einzelnen Neuronen werden nicht beeinflusst. Hierbei wird an dem Reizort die gemäß Abschnitt 3.3 bearbeitete neuronale Aktivität verwendet. Die überraschenderweise eintretende Entkopplung und/oder Desynchronisation wird durch die Interaktion zwischen den Neuronen im getriebenen Areal unterstützt. Man nutzt hierbei einen Wirkmechanismus aus, welcher für die krankhafte Synchronisation verantwortlich ist. Man nutzt anschaulich gesprochen die Energie des zu beeinflussenden Systems aus, um mit minimalem Eingriff einen therapeutischen Effekt zu erzielen. Die besten Ergebnisse werden erzielt, wenn die Stimulationsreize verwendet werden, die aus den Stimulationssignalen erzeugt werden, deren Zeitverzögerungen der ganzzahligen Vielfachen der halben Stimulationsperiode $T$ entsprechen. Die Stimulationsperiode $T$ approximiert die Periode der pathologischen Aktivität. Es werden aber auch noch Behandlungserfolge erzielt, wenn die Zeitverzögerungen der über die Elektrode 2 abgegebenen Reize andere Zeitverzögerungen beinhalten. In so einem Fall wird beispielweise zumindest eine teilweise Entkopplung und/oder Desynchronisation herbeigeführt. Die Behandlungsergebnisse werden aber umso besser, je mehr die gewählten Zeitverzögerungen Vielfachen der halben Periode der pathologischen Aktivität nahe kommen.

## 2 Definition der Begriffe

Neuronale Aktivität:

Die Beschreibung des Mechanismus der erfindungsgemäßen Vorrichtung ist wesentlich gestützt auf den Begriff der neuronalen Aktivität. Die neuronale Aktivität der zu entkoppelnden und/oder der zu desynchronisierenden Neuronenpopulation (siehe Begriffe der treibenden und getriebenen Populationen) wird gemessen, gespeichert und gemäß Abschnitt 3.3 bearbeitet und als Feedback-Stimulationssignal verwendet, wodurch die erfindungsgemäße selbstregulierende Bedarfssteuerung realisiert wird. Im Folgenden wird unter der gemessenen neuronalen Aktivität der zu entkoppelnden und/oder der zu desynchronisierenden Neuronenpopulation ein Signal verstanden, welches die zeitliche Entwicklung der Aktivität der zu entkoppelnden und/oder der zu desynchronisierenden Neuronenpopulation wiedergibt. Beispielsweise können lokale Feldpotentiale die zeitliche Entwicklung der Aktivität der zu entkoppelnden und/oder der zu desynchronisierenden Neuronenpopulation wiedergeben. Die neuronale Aktivität kann vorzugsweise direkt im zu entkoppelnden und/oder im zu desynchronisierenden Areal gemessen werden, es kann aber auch eine der neuronalen Aktivität des zu entkoppelnden und/oder des zu desynchronisierenden Areals zugeordnete Aktivität beispielsweise eines anderen Hirnareals, hier zum Beispiel dem Motorkortex und/oder dem prämotorischen Kortex, oder die Aktivität einer durch das zu entkoppelnde und/oder das zu desynchronisierenden Areal kontrollierte Muskelgruppe gemessen werden. In einer weiteren Ausführungsform der erfindungsgemäßen Vorrichtung können neuronale Aktivitäten, an verschiednen Orten gemessen und kombiniert werden, um eine hinreichende Repräsentation der neuronalen Aktivität der zu entkoppelnde und/oder der zu desynchronisierenden Neuronenpopulation zu erhalten. Auch diese der neuronalen Aktivität des zu entkoppelnden und/oder des zu desynchronisierenden Areals zugeordnete Größen werden im Folgenden als neuronale Aktivität bezeichnet und sind von diesem Begriff umfasst.

Rhythmus:

Unter einem Rhythmus wird die rhythmische, also annähernd periodische neuronale Aktivität verstanden, die sich in Folge einer krankhaft übersteigerten synchronen Aktivität von Nervenzellen ergeben kann. Ein Rhythmus kann kurzzeitig auftretend oder lang anhaltend sein.

Periode:

Ein zentraler Begriff für die erfindungsgemäße Vorrichtung ist die Periode der rhythmischen neuronalen Aktivität, die als zeitliche Referenz für die Applikation der Stimulationsreize dient. Durch Anpassung der Stimulationsperiode $T,$ wie beispielsweise in Abschnitt 7.2.1 beschrieben, wird vorzugsweise bewirkt, dass die Periode der rhythmischen neuronalen Aktivität mit der Stimulationsperiode $T$ übereinstimmt.

Treibende Population:

Unter der treibenden Population wird die Nervenzellpopulation verstanden, welche die pathologisch synchrone neuronale Aktivität generiert oder die pathologisch synchrone Aktivität eines untergeordneten Areals wiedergibt. Die treibende Population kann die pathologisch synchrone Aktivität an die getriebene Population weiterleiten (Fig.4). Der krankhafte Rhythmus der treibenden Neuronenpopulation wird (1) unter Beteiligung im wesentlichen der gesamten treibenden Neuronenpopulation hervorrufen und/oder (2) in einem Teil der treibenden Neuronenpopulation hervorrufen und/oder (3) in einer dritten, von der treibenden und getriebenen Neuronenpopulationen verschiedenen Neuronenpopulation, welche die treibende Neuronenpopulation treibt, hervorrufen.

Im Fall (3) ist die treibende Neuronenpopulation selbst eine getriebene Neuronenpopulation. Die treibende Neuronenpopulation wird auch als zu desynchronisierende Population oder zu desynchronisierendes Areal bezeichnet. Die treibende Nervenzellpopulation ist nicht an anatomische Grenzen gebunden. Vielmehr kann darunter auch mindestens eine Komponente, bestehend aus der Gruppe von:

- mindestens einem Teil von mindestens einem anatomischen Areal,
- mindestens einem vollständigen anatomischen Areal, verstanden werden.

Getriebene Population:

Unter der getriebenen Population wird die Nervenzellpopulation verstanden, die durch die treibende Population direkt oder indirekt beeinflusst wird (Fig.4). Direkte Beeinflussung bedeutet eine Beeinflussung über Fasern, welche direkt - d.h. ohne Zwischenschaltung einer weiteren Population - die beiden Populationen verbinden. Indirekte Beeinflussung bedeutet eine Beeinflussung über mindestens eine zwischengeschaltete Population. Die Nervenzellpopulation, welche durch die treibende Population beeinflusst wird, wird auch als zu entkoppelnde Neuronenpopulation oder als zu entkoppelndes Areal bezeichnet. Das zu entkoppelnde Areal ist nicht an anatomische Grenzen gebunden. Vielmehr kann darunter auch mindestens eine Komponente, bestehend aus der Gruppe von:

- mindestens einem Teil von mindestens einem anatomischen Areal,
- mindestens einem vollständigen anatomischen Areal, verstanden werden.

Als ein Beispiel einer treibenden Neuronenpopulation kann die Verbindung der Areale Nucleus subthalamicus - Globus pallidus exterior dienen, die krankheitsbedingt als Schrittmacher fungieren und eine pathologisch rhythmische synchrone Aktivität generieren können. Die generierte synchrone Aktivität steuert die neuronale Aktivität des Großhirnareales, z. B. des Motorkortexes, der hier als getriebene Population bezeichnet werden kann und weiter mit Muskeln verbunden ist und deren Aktivität kontrolliert.

Entkoppelnde Stimulation:

Unter einer entkoppelnden Stimulation in Sinne der Erfindung wird eine Stimulation verstanden, die den krankhaft antreibenden Effekt der treibenden Neuronenpopulation auf die getriebene Neuronenpopulation soweit minimiert, dass er funktionell - das heißt für die Ausprägung der Symptome - keine Rolle mehr spielt.

Zielpopulation:

Im Folgenden wird unter der Zielpopulation die unmittelbar durch eine implantierte Stimulationselektrode stimulierte Nervenzellpopulation verstanden. Eine Zielpopulation wird durch eine in ihr oder nahe bei ihr implantierte Elektrode direkt stimuliert. Die zu entkoppelnde und/oder zu desynchronisierende Populationen werden entweder direkt oder indirekt stimuliert.

Direkte Stimulation:

In diesem Fall befindet sich die Stimulationselektrode 2 unmittelbar in dem zu entkoppelnden oder in dem zu desynchronisierenden Areal. Diese Elektrode 2 beeinflusst dabei die Zielpopulation, welche sich in dem zu entkoppelnden oder in dem zu desynchronisierenden Areal befindet.

Indirekte Stimulation:

In diesem Fall wird das zu entkoppelnde oder das zu desynchronisierende Areal mittels der Stimulationselektrode 2 nicht direkt stimuliert. Vielmehr wird über die Elektrode 2 eine Zielpopulation oder ein Faserbündel, welche mit dem zu entkoppelnden oder dem zu desynchronisierenden Areal funktionell eng verbunden sind, stimuliert. Hierbei wird der Stimulationseffekt auf das zu entkoppelnde oder das zu desynchronisierende Areal vorzugsweise über anatomische Verbindungen fortgeleitet. Für die indirekte Stimulation soll als Oberbegriff für Zielpopulation und Faserbündel der Begriff Zielareal eingeführt werden. Von dem Begriff Zielareal sollen im Folgenden die mit dem zu entkoppelnden oder dem zu desynchronisierenden Areal funktionell eng verbundene Neuronenpopulation und das verbindende Faserbündel verstanden werden, die unmittelbar durch die implantierte Elektrode 2 stimuliert werden.

Zeitverzögerung:

Durch die erfindungsgemäße Vorrichtung werden Signale an die Stimulationselektrode 2 weitergegeben, welche der gemäß Abschnitt 3.2 gemessenen und gegebenenfalls bearbeiteten neuronalen Aktivität (= Feedback-Stimulationssignal) zu einem früheren Zeitpunkt entsprechen können. Diese zeitliche Verschiebung wird im folgenden Zeitverzögerung genannt und stellt einen wichtigen, im Zusammenhang mit der Stimulationsperiode $T$, die der Periode rhythmischen neuronalen Aktivität entspricht, stehenden, Stimulationsparameter dar.

Feedback-Stimulationssignal:

Unter dem Feedback-Stimulationssignal oder Stimulationssignal wird das Signal verstanden, welches die gemessene und bearbeitete neuronale Aktivität darstellt und als Grundlage für die Stimulationsreize dient. Die Bearbeitungsschritte können beispielsweise wie in Abschnitt 3.3 beschrieben durchgeführt werden. Das Stimulationssignal wird aus der bearbeiteten neuronalen Aktivität zusammengesetzt und zur Stimulation des zu entkoppelnden oder des zu desynchronisierenden Hirnareals herangezogen. Zur Bildung des Feedback-Stimulationssignals kann es notwenig sein, Messsignale durch mehrfache, möglicherweise voneinander unabhängige Bearbeitungsschritte mit unterschiedlichen Bearbeitungsparametern (insbesondere unterschiedlichen Zeitverzögerungen) zu erstellen, die dann zur Bildung des eigentlichen Stimulationssignals z.B. addiert und/oder multipliziert und/oder dividiert und/oder subtrahiert und/oder mittels anderer nichtlinearer Funktionen berechnet werden. Aus den Feedback-Stimulationssignalen werden Stimulationsreize erzeugt und danach mittels der Elektrode auf die Zielpopulation appliziert.

Resultierender Stimulationseinfluss

Unter dem resultierenden Stimulationseinfluss auf die zu entkoppelnde oder die zu desynchronisierende Neuronenpopulationen wird die Summe der auf die zu entkoppelnde und/oder zu desynchronisierende Population applizierten externen Kräfte verstanden. Gemäß Abschnitt 3.1, wird bei einer Ausführungsform der erfindungsgemäßen Vorrichtung die getriebene Neuronenpopulation von der treibenden Neuronenpopulation mittels direkter oder indirekter Stimulation entkoppelt. In diesem Fall ist der resultierende Stimulationseinfluss auf die zu entkoppelnde Population die Summe des Stimulationssignals und der treibenden Kraft der Kopplung mit der treibenden Population. Bei einer anderer Ausführungsform der erfindungsgemäßen Vorrichtung wird die zu desynchronisierende, treibende Population mittels der Stimulation desynchronisiert. Hier ist der resultierende Stimulationseinfluss auf die zu desynchronisierende Population nur das Stimulationssignal. Aufgrund der in Abschnitt 5 beschriebenen selbstregulierende Bedarfsteuerung wird die Amplitude des resultierenden Stimulationseinflusses auf die zu entkoppelnde oder die zu desynchronisierende Neuronenpopulation nach erfolgreicher Entkopplung und/oder Desynchronisation selbständig minimiert.

**3 Stimulationsverfahren und Reizform**

**3.1 Entkoppelndes und desynchronisierendes Verfahren**

Eine pathologisch synchrone Neuronenpopulation in einem Hirnareal kann durch rhythmische Aktivität als treibende Kraft auf eine andere nachgeschaltete Neuronenpopulation einwirken. Damit kann sich zwischen den Populationen ein Interarntionsschema in der Form "treibende Population - getriebene Population" ergeben, wie in Fig.4 schematisch dargestellt wird. Wenn die treibende Kraft stark genug ist, wird die getriebene Neuronenpopulation auch synchronisiert, was die krankhafte Symptomatik hervorrufen kann. Dies tritt auf, wenn die getriebene Population die Muskeln ansteuert, wie dies beim prämotorischer Kortex oder Motorkortex der Fall ist.

Wie in Abschnitt 1 beschrieben, ist es das Ziel der erfindungsgemäßen Vorrichtung und der erfindungsgemäßen Stimulationsverfahren, die pathologisch synchrone neuronale Aktivität zu desynchronisieren, was die Unterdrückung der Symptomatik erwarten lässt. Dazu wird, im Falle des entkoppelnden Stimulationsmodus, die getriebene Neuronenpopulation 2 von der treibenden Population 1 entkoppelt und desynchronisiert, oder, bei desynchronisierenden Stimulationsmodus, die treibende Neuronenpopulation 1 desynchronisiert.

Beim entkoppelnden Stimulationsmodus wird die getriebene Neuronenpopulation 2 mittels einer Stimulationselektrode direkt oder indirekt gemäß Abschnitten 3.4 und 4.1 stimuliert. Die Stimulation bewirkt eine Entkopplung der Neuronenpopulation 2 von der treibenden Neuronenpopulation 1 wodurch es zu einer Desynchronisation der Population 2 kommt.

Beim desynchronisierenden Stimulationsmodus wird die treibende Neuronenpopulation 1 mittels einer Stimulationselektrode direkt oder indirekt stimuliert. Mittels dieser Stimulation wird Population 1 desynchronisiert, so dass ihre treibende Kraft auf Population 2 entfällt. Die Letztere wird dann auch desynchronisiert, wodurch die pathologischen Symptome unterdrücken werden. Falls sich Population 2 selbst synchronisiert, muss sie wie eine treibende Neuronenpopulation direkt desynchronisiert werden.

Bei beiden oben erwähnten Stimulationsverfahren erfolgt gemäß Abschnitt 5 eine selbstregulierende Bedarfssteuerung des Stimulationssignals, wobei der resultierende Stimulationseinfluss auf die stimulierte Neuronenpopulation selbständig minimiert wird. Gemäß Abschnitt 2 ist im entkoppelnden Stimulationsmodus der resultierende Stimulationseinfluss auf die getriebene Neuronenpopulation die Summe des Stimulationssignals und der treibenden Kraft der treibenden Population. Bei dem desynchronisierenden Stimulationsverfahren ist der resultierende Stimulationseinfluss auf die treibende Neuronenpopulation ausschließlich der Einfluss des Stimulationssignals.

Im Folgenden wird beispielhaft eine Ausführungsform der erfindungsgemäßen Vorrichtung beschrieben, nämlich der entkoppelnde Stimulationsmodus, bei dem die zu entkoppelnde Neuronenpopulation mittels direkter oder indirekter Stimulation von der treibenden Neuronenpopulation entkoppelt wird. Die weiteren Ausführungsformen der erfindungsgemäßen Vorrichtung werden in Abschnitt 8 beschrieben.

## 3.2 Messung der neuronalen Aktivität

Der zeitliche Verlauf der neuronalen Aktivität des zu entkoppelnden und/oder des treibenden Areals kann über die Sensoren 3 direkt und/oder indirekt gemessen werden.

Bei indirekter Messung wird über mindestens einen der Sensoren 3 der zeitliche Verlauf der Aktivität einer durch das zu entkoppelnde und/oder das treibende Areal beeinflussten Muskelgruppe und/oder der zeitliche Verlauf der Aktivität einer dem zu entkoppelnden und/oder dem treibenden Areal zugeordneten Neuronenpopulation gemessen.

Die Sensoren 3 (siehe Figur 1) befinden sich im Gehirn und/oder außerhalb des Gehirns. Im Gehirn sind sie im zu entkoppelnden und/oder im treibenden Areal und/oder in mindestens einem anderen, funktionell damit in Verbindung stehenden Areal positioniert. Außerhalb des Gehirns befinden sich die Sensoren 3 an Körperteilen, die mit der krankhaft synchronisierten neuronalen Aktivität in Verbindung stehen, z.B. als Elektroden an einem zitternden Muskel. Die gemessenen Signale der neuronalen Aktivität der Neuronenpopulationen, beispielsweise der muskulären Aktivität (welche auch als neuronale Aktivität bezeichnet wird, siehe Abschnitt 2), werden in einer Einheit zur Signalverarbeitung 4 bearbeitet und gespeichert. Die Messung, Bearbeitung und Speicherung kann dabei permanent oder in zeitlich diskreten Abständen erfolgen. Im letzteren Fall werden die Dauer und/oder die Abstände der diskreten Messintervalle durch einen deterministischen und/oder stochastischen Algorithmus bestimmt.

## 3.3 Bearbeitung der neuronalen Messsignale

Die in der Einheit zur Signalverarbeitung 4 gespeicherten Messsignale werden sodann bearbeitet, um als Feedback-Stimulationssignale zur Verfügung zu stehen. Folgende Bearbeitungsschritte können angewendet werden:

1. Die gemessene neuronale Aktivität kann gefiltert werden, z.B. kann eine Bandpassfilterung der neuronalen Aktivität vorgenommen werden. Die Filterung kann notwenig sein, falls über Sensor 3 neben der krankheitsspezifischen Aktivität zusätzlich noch nicht krankheitsspezifische Aktivität, zum Beispiel aus anderen Neuronenpopulationen, gemessen wird. Da die krankheitsspezifische Aktivität typischerweise in einem Frequenzbereich auftritt, der von dem Frequenzbereich der nichtkrankheitsspezifischen Aktivität verschieden ist, wird in diesem Fall vorzugsweise eine Ermittlung der Aktivität im krankheitsspezifischen Frequenzbereich durchgeführt. Dies wird beispielsweise durch eine Frequenzanalyse realisiert. Ebenso kann es notwendig sein eine Wavelet-Analyse und/oder eine Hilbert-Transformation und/oder eine Filterung in zeitlicher Domäne durchzuführen.

2. Wird über mehrere Sensoren 3 die neuronale Aktivität der zu entkoppelnden und/oder der zu desynchronisierenden Neuronenpopulation gemessen, so kann eine lineare und/oder nichtlineare Kombination der gemessenen neuronalen Aktivitäten durchgeführt werden. Beispielsweise werden die gemessenen neuronalen Signale untereinander oder mit sich selbst multipliziert, dividiert, addiert und/oder subtrahiert und/oder mittels anderer nichtlinearer Funktionen transformiert.

3. Die gemessene neuronale Aktivität wird zeitlich verzögert. Die hierzu verwendeten Zeitverzögerungen werden in den Abschnitten 3.4 definiert und berücksichtigen auch, gemäß Abschnitt 7.3, die Lage der Stimulationselektrode bezüglich der zu entkoppelnden Neuronenpopulation. Darüber hinaus können die Zeitverzögerungen vorzugsweise während der Stimulation gemäß Abschnitt 7.2.1 und 7.2.2 angepasst werden.

4. Die gemessene neuronale Aktivität wird verstärkt. Typischerweise ist die gemessene neuronale Aktivität um einige Größenordnungen geringer, als die Stimulationsamplituden, die erfahrungsgemäß zu einer Stimulationswirkung führen. Daher ist eine Verstärkung, welche während der Stimulation gemäß Abschnitt 7.2.3 angepasst werden kann, durchzuführen.

5. Die gemessene neuronale Aktivität wird zeitlich kodiert. Da Signale mit hohen Gradienten eine große Wirkung auf die neuronale Dynamik haben, wird die gemessene neuronale Aktivität beispielsweise in Form von Pulszügen bzw. Niedrig- oder Hochfrequenzpulszüge bestehend aus kurzen Rechteckpulsen kodiert. Es können zur Steigerung der Stimulationswirkung auch andere Kodierungsverfahren verwendet werden.

6. Die Polarität der neuronalen Aktivität wird geändert.

7. Die neuronale Aktivität wird linear und/oder nichtlinear transformiert. Dies kann z.B. mit Hilfe der Hilbert- und/oder Fourier- und/oder Wavelet-Transformation geschehen.

8. Die maximale Amplitude des Stimulationssignals wird beschränkt.

9. Die gemessene neuronale Aktivität wird dahingehend transformiert, so dass Stimulationssignale entstehen, deren Nettoladungseintrag im Wesentlichen Null ist.

10. Die gemessene neuronale Aktivität wird direkt als Feedback-Stimulationssignal verwendet.

Die bearbeitete neuronale Aktivität, d.h. das Feedback-Stimulationssignal, wird durch die Anwendung mindestens einer Komponente der oben genannten Bearbeitungsschritte ermittelt.

Beispielsweise können die Stimulationssignale aus der gemessenen neuronalen Aktivität mit immer gleichen Bearbeitungsschritten erzeugten werden. Es kann auch der Satz der Behandlungsschritte und/oder deren Parameter durch einen deterministischen und/oder stochastischen und/oder kombiniert stochastisch/deterministischen Algorithmus zeitlich variiert werden.

### 3.4 Form des Stimulationsreizes

Unter einem Stimulationsreiz wird ein Reiz verstanden, der über die Elektrode 2 appliziert wird und in einem Zeitintervall wirkt. Zur Bildung eines Stimulationsreizes werden die Feedback-Stimulationssignale, dass heißt, die gemäß Abschnitt 3.3 bearbeitete neuronale Aktivität, verwendet. Um Stimulationsreize zu generieren, werden die Stimulationssignale beispielsweise untereinander und/oder mit sich selbst multipliziert, dividiert, addiert und/oder subtrahiert und/oder mittels anderer nichtlinearer Funktionen transformiert.

Die bei der Bearbeitung der neuronalen Aktivität verwendeten Zeitverzögerungen werden beispielsweise als Bruchteile der Periode der oszillatorischen, zu entkoppelnden und/oder treibenden neuronalen Aktivität angegeben und betragen vorzugsweise im wesentlichen ein Vielfaches eines N-tel der Periode wobei N eine kleine ganze Zahl, zum Beispiel 2, ist. Die Zeitverzögerungen der Stimulationssignale können auch z.B. größer als die Stimulationsperiode $T$ gewählt werden. Die erfindungsgemäße Vorrichtung bietet auch die Möglichkeit mehrere, vorzugsweise unterschiedliche Zeitverzögerungen zur Bildung des Stimulationsreizes heran zu ziehen. Die resultierenden zeitverzögerten Feedback-Stimulationssignale können linear- und/oder nichtlinear zu einem Stimulationsreiz kombiniert werden.

Hierzu verfügt die erfindungsgemäße Vorrichtung über Mittel, welche den beschriebenen elektrischen Stimulationsreiz in der beschriebenen Art appliziert. Die Mittel sind die Elektrode 2, eine Steuerung 4, welche Steuersignale an die Elektrode 2 für die Abgabe dieser Reize abgibt. Des weiteren Sensoren 3 und die Einheit zur Signalverarbeitung 4, welche die neuronale Aktivität aufnimmt und für die weitere Verwendung als Stimulationsreiz vorbereitet. Vorzugsweise wird ein Stimulationsreiz generiert, dessen Nettoladungseintrag im Wesentlichen Null ist.

Beispielsweise kann die Elektrode 2 mit dem gleichen Stimulationsreiz, in Form der gleichen bearbeiteten neuronalen Aktivität gemäß Abschnitt 3.3 angesteuert werden. Es kann auch die Elektrode 2 mit unterschiedlichen Stimulationssignalen und/oder Kombinationen der Stimulationssignale und/oder mittels unterschiedlicher Transformationen und/oder Kombinationen der Stimulationssignale generiert werden, angesteuert werden.

Es können die Reihenfolge und/oder die Art und/oder der Energieeintrag und/oder die Parameter der Reize mit einem deterministischen und/oder stochastischen und/oder kombiniert stochastisch/deterministischen Algorithmus ermittelt werden.

Es können die in den Bearbeitungsschritten, siehe Abschnitt 3.3, verwendeten Zeitverzögerungen und/oder Polarität und/oder Applikationsdauer und/oder Intensität des Stimulationsreizes systematisch oder zufallsgesteuert, das heißt, gemäß einer deterministischen oder stochastischen Regel, variiert werden. Hierzu verfügt die erfindungsgemäße Vorrichtung über eine Steuerung, die so programmiert ist, dass sie die Zeitverzögerungen und/oder die Polarität und/oder die Applikationsdauer und/oder die Intensität der Bearbeitungsschritte des Stimulationsreizes deterministisch und/oder stochastisch ansteuert.

Durch Variation der Zeitverzögerungen und/oder der Polarität und/oder der Applikationsdauer und/oder der Intensität innerhalb der Bearbeitungsschritte des Stimulationssignals kann Adaptationsvorgängen in den Neuronenpopulationen, die eine Erhöhung der Stimulationsintensität zum Erreichen derselben therapeutischen Wirkung bewirken, vorgebeugt werden.

### 4 Räumliche Anordnung der Elektrode und Sensoren

#### 4.1 Stimulationselektrode

Es wird vorzugsweise eine Elektrode 2 zur Stimulation verwendet.

Für den Fall, dass die Elektrode 2 in der zu entkoppelnde Nervenzellpopulation positioniert ist, soll die Elektrode vorzugsweise so angeordnet sein, dass mit der Elektrode die gesamte zu entkoppelnde Nervenzellpopulation stimuliert werden kann. Dies kann mit geometrischer Positionierung der Elektrode realisiert werden. Beispielsweise kann die Elektrode 2 im Zentrum des zu entkoppelnden Areals positioniert werden.

Für den Fall, dass die Elektrode 2 nicht in der zu entkoppelnden Nervenzellpopulation positioniert ist, wird bei dieser Stimulationsform in einem von dem zu entkoppelnden Areal verschiedenen Zielareal stimuliert. Hierbei kann die indirekte Stimulation durch Stimulation einer von der zu entkoppelnden Nervenzellpopulation verschiedenen Neuronenpopulation und/oder durch Stimulation eines mit der zu entkoppelnden Nervenzellpopulation verbundenen Faserbündels erfolgen.

#### 4.2 Anzahl der Sensoren

Der Mechanismus der erfindungsgemäßen Vorrichtung besteht im wesentlichen darin, dass - wie in Abschnitt 1 und 3 beschrieben - die gemessenen und bearbeiteten neuronalen Aktivitäten der zu entkoppelnden und/oder der treibenden Neuronenpopulation wieder als Stimulation appliziert werden. Die Sensoren 3 sind eine der wichtigsten Komponenten der erfindungsgemäßen Vorrichtung und können, wie in Abschnitt 3.2 beschrieben, entweder außerhalb der zu entkoppelnden und der treibenden Neuronenpopulation oder vorzugsweise direkt in der zu entkoppelnden und/oder der treibenden Neuronenpopulation positioniert sein. Vorzugsweise wird nur ein Sensor 3 verwendet, um die Aktivität der zu entkoppelnden und/oder der treibenden Neuronenpopulation zu detektieren. Dadurch wird die Anzahl der zu implantierenden Sensoren möglichst gering gehalten, um unnötigen Gewebsschädigungen und vor allem einer Hirnblutung während der Implantation vorzubeugen. Es können aber auch beispielsweise zwei oder mehr Sensoren eingesetzt werden, um die neuronale Aktivität der zu entkoppelnden Neuronenpopulation und/oder der treibenden Neuronenpopulation als Kombination der gemessenen Aktivitäten viel vollständiger zu rekonstruieren.

Des Weiteren werden mögliche durch die Implantation verursachte Hirnschädigungen weiter reduziert oder vermieden, und der Stimulationseffekt verbessert, indem mindestens ein Sensor 3 und Stimulationselektrode 2 in einer zu implantierenden Elektrode kombiniert werden.

Für den Fall, dass die Sensoren 3 alle in der zu entkoppelnden und/oder der treibenden Nervenzellpopulation positioniert sind, sollen die Sensoren 3 vorzugsweise so angeordnet sein, dass mit den Sensoren ein großer Teil der zu entkoppelnden und/oder der treibenden Nervenzellpopulation erfasst werden kann. Dies kann mit geometrischer Anordnung der Sensoren im Hinblick auf das zu entkoppelnde und/oder das treibende Gewebe realisiert werden. Im Falle einer Anordnung mit nur einem Sensor 3 kann dieser beispielsweise im Zentrum des Gewebes lokalisiert werden. Im Falle von Anordnungen mit mehreren Sensoren können die Sensoren beispielsweise in einer symmetrischen Weise angeordnet werden.

Für den Fall, dass mindestens einer der Sensoren 3 nicht in der zu entkoppelnden und der treibenden Nervenzellpopulation positioniert ist, wird bei dieser Form der Aktivitätsmessung eine der neuronalen Aktivität der zu entkoppelnden und/oder der treibenden Neuronenpopulation zugeordnete Aktivität in mindestens einem, von dem zu entkoppelnden und dem treibenden Areal, verschiedenen Areal gemessen. Hierbei kann, wie in Abschnitt 3.2 beschrieben, die indirekte Messung durch die Messung der Aktivität einer von der zu entkoppelnden und der treibenden Nervenzellpopulation verschiedenen Neuronenpopulation und/oder eines Faserbündels und/oder eines Körperteils erfolgen, welches mit der zu entkoppelnden/der treibenden Nervenzellpopulation verbunden ist.

### 5 Selbstregulierende Bedarfssteuerung des Stimulationssignals

Eine der wichtigsten Eigenschaften des Mechanismus der erfindungsgemäßen Vorrichtung ist eine selbstregulierende Bedarfssteuerung des Stimulationssignals. Die beschriebene Selbstregulierung erfolgt dadurch, dass der Stimulationsreize aus der bearbeiteten neuronalen Aktivität bestehen. Im Fall einer stärkeren synchronen Aktivität im zu entkoppelnden Areal und/oder einer Kopplung mit der treibenden Population des zu entkoppelnden Areals ist, wie dem Fachmann bekannt, eine große Varianz der gemessenen neuronalen Aktivität zu erwarten. Dies führt direkt zu einer erfindungsgemäß zeitverzögerten, Stimulation mit einer erhöhten Stimulationsamplitude. Die Kraft des applizierten Stimulationssignals kompensiert erfindungsgemäß und anschaulich gesprochen die Kraft der internen Synchronisation und/oder der Kopplung mit der treibenden Population des zu entkoppelnden Areals, so dass eine Entkopplung und Desynchronisation der zu entkoppelnden Population resultiert. Dadurch wird die Amplitude des resultierenden Stimulationseinflusses auf die zu entkoppelnde Population, d.h. der Summe von Stimulation und Kopplung, eigenständig minimiert. Nach Erreichen einer Entkopplung und Desynchronisation wird eine neuronale Aktivität geringer Varianz erwartet, wodurch die Stimulationssignale direkt beeinflusst und selbstständig angepasst werden. Tritt wieder eine erneute Kopplung und/oder eine Resynchronisation ein, so trägt die erfindungsgemäße Vorrichtung dem gesteigerten Bedarf nach entkoppelnder und/oder desynchronisierender Stimulation automatisch Rechnung, indem die größere Varianz der neuronalen Aktivität zur Bildung eines stärkeren Stimulationsreizes führt. Dies stellt eine selbstregulierende Bedarfssteuerung der erfindungsgemäßen Vorrichtung dar.

Die der selbstregulierenden Bedarfssteuerung zugrunde liegende Mechanismus wirkt in allen im Folgenden näher beschriebenen Ausführungsformen der erfindungsgemäßen Vorrichtung.

## 6 Steuerung der Reizapplikation

Unter der zeitlichen Steuerung der Reizapplikation wird eine vorzugsweise im Voraus programmierte Ausführungsform der erfindungsgemäßen Vorrichtung verstanden, wobei der Stimulationsreiz auf eine bestimmte Art mittels der Stimulatoreinheit 8 appliziert wird. Die Varianten der zeitlichen Steuerung der Reizapplikation sind permanente, wiederkehrende und bedarfsgesteuerte Reizapplikation. Zusätzlich kann eine manuelle Bedarfssteuerung, zum Beispiel für eine durch den Patienten oder Arzt durchgeführte Reizapplikation, implementiert werden.

### 6.1 Permanente Reizapplikation

Bei der permanenten Reizapplikation verfügt die erfindungsgemäße Vorrichtung über eine Steuerung, die so programmiert ist, dass sie an der Elektrode 2 eine fortdauernde Applikation des Stimulationsreizes vornimmt. Die permanente Reizapplikation stellt die einfachste und leicht zu realisierende Ausführungsform der erfindungsgemäßen Vorrichtung dar. Gleichzeitig erfolgt aufgrund der in Abschnitt 5 beschriebenen erfindungsgemäßen selbstregulierenden Bedarfssteuerung eine gute entkoppelnde und desynchronisierende Wirkung der permanenten Stimulation bei geringem Energieeintrag in die zu entkoppelnde Population.

Während der permanenten Reizapplikation kann eine Anpassung der Intensitätparameter gemäß Abschnitt 7.2.3 stattfinden.

Ebenso kann eine Anpassung der Zeitparameter - Stimulationsperiode $T$ und/oder Zeitverzögerungen - während der permanenten Stimulation gemäß Abschnitt 7.2.1 und 7.2.2 in Kombination mit einer Anpassung der Stimulationsintensität oder unabhängig davon erfolgen.

### 6.2 Wiederkehrende Reizapplikation

Bei der wiederkehrenden Reizapplikation verfügt die erfindungsgemäße Vorrichtung über eine Steuerung, die so programmiert ist, dass sie an der Elektrode 2 eine Applikation des Stimulationsreizes nur während bestimmter Zeitintervalle vornimmt. Außerhalb dieser Zeitintervalle erfolgt keine Stimulation.

Bei der wiederkehrenden Reizapplikation können der Stimulationsreiz zeitlich streng periodisch oder zeitlich nicht periodisch verabreicht werden. In dieser Ausführungsform verfügt die erfindungsgemäße Vorrichtung über eine Steuerung, die so programmiert ist, dass sie die Zeitabstände zwischen den Stimulationsintervallen und/oder die Dauer der Intervalle periodisch und/oder nicht periodisch kontrolliert. Eine zeitlich nicht periodische Abfolge des Stimulationsreizes kann durch einen stochastischen und/oder deterministischen und/oder kombiniert stochastischen/deterministischen Algorithmus generiert werden, um den gewünschten entkoppelten und desynchronisierten Zustand der zu entkoppelnden Population zu erreichen. Analog wird im Folgenden unter einer Kombination deterministischen und stochastischen Gesetzmäßigkeiten ein funktionaler Zusammenhang verstanden, bei dem deterministische und stochastische Terme funktionell, z. B. durch Addition und/oder Multiplikation miteinander verbunden sind.

Die Stimulations- und Messintervalle können überlappend oder zeitgleich oder zeitlich getrennt angeordnet sein.

Während der wiederkehrenden Reizapplikation kann eine Anpassung der Intensitätsparameter gemäß Abschnitt 7.2.3 stattfinden. Ebenso kann eine Anpassung der Zeitparameter - Stimulationsperiode T und/oder Zeitverzögerungen - während der wiederkehrenden Stimulation gemäß Abschnitt 7.2.1 und 7.2.2 in Kombination mit einer Anpassung der Stimulationsintensität oder unabhängig davon erfolgen.

### 6.3 Bedarfsgesteuerte Reizapplikation

Bei der bedarfsgesteuerten Reizapplikation verfügt die erfindungsgemäße Vorrichtung über eine Steuerung, die so programmiert ist, dass sie das An- und Abschalten des Stimulationsreizes entsprechend der bestimmten Zustände der zu entkoppelnden und/oder der treibenden Neuronenpopulation vornimmt. Dazu verwendet die Steuereinheit 4 die Messsignale und/oder die Stimulationssignale zur Detektion eines pathologischen Merkmals. Das Anschalten der Stimulation erfolgt beispielsweise wie im Folgenden beschreiben.

Über Sensor 3 wird die Aktivität der zu entkoppelnden und/oder der treibenden Neuronenpopulation gemessen. Die neuronale Aktivität wird an die Einheit 4 zur Signalverarbeitung und/oder Regelung weitergeleitet, die unter anderem als Mittel zum Erkennen eines pathologischen Merkmals füngiert. Sobald die Einheit 4 zur Signalverarbeitung und/oder Regelung in der neuronalen Aktivität ein pathologisches Merkmal erkennt, wird die Applikation eines Stimulationsreizes gestartet. Vorzugsweise wird, sobald das pathologische Merkmal aufgrund der Wirkung der applizierten Stimulation verschwindet, die Stimulation abgeschaltet. Die erfindungsgemäße Vorrichtung umfasst daher in einer möglichen Ausführungsform als Einheit 4 zur Signalverarbeitung und/oder Steuerung/Regelung einen Rechner, welcher einen Datenträger beinhaltet, der die Daten des Krankheitsbildes trägt und mit den Messdaten vergleicht. Unter den Daten des Krankheitsbildes werden für die Stimulation relevante Parameter und Messgrößen verstanden, zum Beispiel die Momentanfrequenz der über Sensor 3 gemessenen neuronale Aktivität, des für die Verfahrensweise der bedarfsgesteuerten Reizapplikation notwendigen Schwellenwerts, die Stimulationsparameter, welche die Reizstärke festlegen. Unter einem pathologischen Merkmal ist beispielsweise eine krankheitsbedingte Synchronisation der zu entkoppelnden/der treibenden Neuronenpopulation zu verstehen und kann durch folgende Eigenschaften der neuronalen Aktivität erkannt werden:

a) Falls über den Sensor 3 ausschließlich bzw. überwiegend die pathologische Aktivität der zu entkoppelnden und/oder der treibenden Neuronenpopulation gemessen wird, wie z.B. bei der in Abschnitt 3.2 und Abschnitt 4.2 beschriebenen direkten Messung, wird die neuronale Aktivität direkt zur Feststellung, ob die Amplitude der neuronalen Aktivität einen Schwellenwert überschreitet, verwendet. Die erfindungsgemäße Vorrichtung ist daher in einer bevorzugten Ausführungsform mit Mitteln zum Erkennen eines dem Schwellenwert entsprechenden Wertes der Amplitude der neuronalen Aktivität ausgestattet. In diesem Fall wird vorzugsweise die neuronale Aktivität selbst und/oder ihr Betrag und/oder ihre Amplitude mit dem Schwellenwert verglichen. Das Mittel zum Erkennen des Schwellenwertes kann bei dieser Ausführungsform so programmiert sein, dass es beispielsweise die neuronale Aktivität selbst und/oder ihr Betrag und/oder ihre Amplitude mit dem Schwellenwert vergleicht. Die Bestimmung der Amplitude erfolgt entweder in einer einfachen Version mittels Bestimmung des Betrags des Signals, und/oder mit Bandpassfilterung und/oder Hilbert-Transformation und/oder Wavelet-Analyse. Die Einheit 4 zur Signalverarbeitung ist in diesem Fall so programmiert, dass sie eine Bestimmung des Betrags des Signals und/oder eine Bandpassfilterung und/oder Hilberttransformation und/oder eine Wavelet-Analyse durchführen kann. Die neuronale Aktivität oder ihr Betrag wird besonders bevorzugt verwendet, da die Berechnung der Amplitude einen deutlich höheren Rechenaufwand bedeutet, und die Bestimmung der Amplitude nicht auf einem einzelnen Messwert der neuronalen Aktivität durchgeführt werden kann, sondern in einem hinreichend großem, dem Fachmann bekannten Zeitintervall durchgeführt werden muss, was die Erkennung des pathologischen Merkmals etwas verzögern kann.

b) Falls über Sensor 3 neben dieser pathologischen Aktivität der zu entkoppelnden und/oder der treibenden Neuronenpopulation zusätzlich noch nicht krankheitsspezifische Aktivität, zum Beispiel aus anderen Neuronenpopulationen, gemessen wird, wie z.B. bei der in den Abschnitten 3.2 und 4.2 beschriebenen indirekten Messung, muss bei der Analyse der neuronalen Aktivität ein weiterer algorithmischer Schritt eingefügt werden. Da die krankheitsspezifische Aktivität typischerweise in einem Frequenzbereich auftritt, der von Frequenzbereich der nicht krankheitsspezifischen Aktivität verschieden ist, genügt es hierzu vorzugsweise eine Abschätzung der Aktivität im krankheitsspezifischen Frequenzbereich durchzuführen. Die Frequenz der krankheitsspezifischen Aktivität wird beispielsweise durch eine Bestimmung der Differenz von aufeinanderfolgenden Triggerpunkten durchgeführt. Triggerpunkte sind Punkte, wie Maxima, Minima, Wendepunkte und Nulldurchgänge. Vorzugsweise wird diese Analyse in einem gleitenden Zeitfenster durchgeführt, wobei der Mittelwert von mehreren zeitlichen Differenzen gebildet wird, wodurch die Stabilität erhöht wird. Alternativ kann die Frequenzschätzung auch mit den dem Fachmann bekannten spektralen Schätzmethoden und anderen Frequenzschätzern wie z. B. mit Hilfe einer Fourieranalyse bestimmt werden. Hierzu verfügt die erfindungsgemäße Vorrichtung in einer besonderen Ausführungsform Mittel zur Abschätzung der Aktivität im krankheitsspezifischen Frequenzbereich, wie spektrale Schätzmethoden, Fourier- und/oder Wavelet-Analyse u.s.w.. Dies wird beispielsweise durch Mittel zum Durchführen einer Frequenzanalyse realisiert. Es kann beispielsweise die spektrale Energie im krankheitsspezifischen Frequenzbereich in einem gleitenden Fenster bestimmt werden. Alternativ kann nach Bandpassfilterung die Amplitude im krankheitsspezifischen Frequenzbereich durch Bestimmung des Maximums des bandpassgefilterten Signals oder durch Bestimmung des Mittelwerts des Betrags des bandpassgefilterten Signals und/oder mit Hilbert-Transformation und/oder mittels Wavelet-Analyse ermittelt werden. Hierzu umfasst die erfindungsgemäße Vorrichtung beispielsweise Mittel zur Bandpassfilterung der Amplitude und Mittel zur Bestimmung des Maximums des bandpassgefilterten Signals und/oder Mittel zur Bestimmung des Mittelwerts des Betrags des bandpassgefilterten Signals und/oder Mittel zur Durchführung einer Hilberttransformation und/oder einer Wavelet-Analyse.

Bei bedarfsgesteuerter Reizapplikation wird beispielsweise immer derselbe Stimulationsreiz verwendet. Vorzugsweise wird die Stimulationsperiode $T$ wie in Abschnitt 7.2.1 beschrieben an die momentane Frequenz der zu entkoppelnden und/oder der zu treibenden Neuronenpopulation angepasst. Es wird dann bei Vorliegen des pathologischen Merkmals ein Reiz mit an die momentane Frequenz angepasster Stimulationsperiode $T$ appliziert. Ebenso können die Zeitverzögerungen gemäß Abschnitt 7.2.2 angepasst werden und/oder die Intensität dieses Reizes bleibt dabei vorzugsweise konstant. Die Intensitätsparameter können aber auch wie in Abschnitt 7.2.3 gemäß des Stimulationseffektes modifiziert werden.

### 6.3.1 Feststellung des Bedarfs

Aus mindestens zwei Gründen gibt es keine eineindeutige Beziehung zwischen der Ausprägung des pathologischen Merkmals und der Ausprägung der krankheitsspezifischen Symptome. Zum einen bedingt die Entfernung von Sensor 3 zu dem zu entkoppelnden und/oder dem treibenden Areal, in welchem die zu messende neuronale Aktivität generiert wird, eine Veränderung der Amplitude im krankheitsspezifischen Frequenzbereich. Zum anderen ist eine bestimmte Ausprägung des krankheitsspezifischen Merkmals, das heißt die Ausprägung der rhythmischen Aktivität im krankheitsspezifischen Frequenzbereich, nicht eineindeutig mit den krankheitsspezifischen Symptomen verbunden. Da der krankheitsspezifische Rhythmus Auswirkungen auf komplexe Nervennetzwerke im Gehirn hat, die typischerweise obendrein nicht einfachen linearen dynamischen Gesetzmäßigkeiten gehorchen, gelten keine eineindeutigen Relationen zwischen krankheitsspezifischem Rhythmus und Ausprägung der Symptome. Wenn zum Beispiel der krankheitsspezifische Rhythmus nicht hinreichend mit der biomechanisch vorgegebenen Eigenfrequenz einer Extremität übereinstimmt, ist der durch den krankheitsspezifischen Rhythmus bedingte Tremor deutlich geringer, als wenn der krankheitsspezifische Rhythmus in Resonanz mit der biomechanisch vorgegebenen Eigenfrequenz der Extremität übereinstimmt.

Die charakteristischen Eigenschaften, wie z.B. die dominierende Frequenz und die Amplitude, der gemessenen neuronalen Aktivität befinden sich in einem dem Fachmann bekannten Erfahrungsbereich. Der Wert der Ausprägung des krankheitsspezifischen Merkmals der über Sensor 3 gemessenen neuronalen Aktivität wird als Schwelle bezeichnet, bei dessen Überschreiten es typischerweise zum Auftreten von Symptomen, zum Beispiel des Tremors, kommt. Die Schwelle ist ein Parameter, der für die Ausführungsform der in Abschnitt 6.3 beschriebenen bedarfsgesteuerten Reizapplikation gewählt werden muss. Die erfindungsgemäße Vorrichtung umfasst daher in Form der Steuereinheit 4 Mittel zum Erkennen eines Schwellenwertes. Mit dem erfindungsgemäßen Verfahren der bedarfsgesteuerten Reizapplikation wird der Vorteil erreicht, dass die Wirksamkeit der erfindungsgemäßen Vorrichtung nicht kritisch von der Wahl der Schwelle abhängt, sondern bezüglich der Wahl der Schwelle eine große Fehlertoleranz gegeben ist, die beispielsweise in einem Bereich von bis zu 50% der maximalen Ausprägung des krankheitsspezifischen Merkmals liegt. Die Wahl der Schwelle wird entweder intraoperativ oder vorzugsweise in den ersten Tagen nach der Operation durch Messung der neuronalen Aktivität über Sensor 3, mit Bestimmung der Ausprägung des krankheitsspezifischen Merkmals und Vergleich mit der Ausprägung der Symptome, z. B. der Stärke des Zitterns, bestimmt.

In einer weniger bevorzugten Ausführungsform der bedarfsgesteuerten Reizapplikation wird als Schwelle ein repräsentativer Wert, zum Beispiel der Mittelwert, eines Kollektivs von bei Patienten gemessenen Schwellenwerten genommen.

In einer bevorzugten Ausführungsform wird die Wahl der Schwelle in im Wesentlichen regelmäßigen Abständen, zum Beispiel im Rahmen von halbjährlichen Kontrollen, überprüft.

In der in den Abschnitten 6.1 und 6.2 beschriebenen Ausführungsformen der permanenten und wiederkehrenden Stimulation mit bedarfsgesteuerter Reizstärke ist keine Schwellenwertdetektion notwendig.

Die oben beschriebenen drei Stimulationsmethoden können vorzugsweise in unterschiedlicher Kombination mit den in Abschnitt 7.2 beschriebenen Methoden zur Anpassung der Stimulationsparameter verwendet werden.

Allen drei Stimulationsmethoden gemeinsam ist die inhärente erfindungsgemäße selbstregulierende Bedarfssteuerung. Die direkte Abhängigkeit der Stimulationssignale von der gemessenen neuronalen Aktivität bedingt eine, in Abschnitt 5 beschriebene, selbstregulierende Bedarfssteuerung, wodurch der Energieeintrag in die zu entkoppelnde Population minimiert wird. Diese selbstregulierende Bedarfssteuerung wirkt unabhängig von der Realisation der in Abschnitt 6.3 beschriebenen zusätzlichen Bedarfssteuerung und der Kalibrierung und Regelung der Parameter, wie sie in Abschnitt 7 beschrieben werden.

### 7 Kalibrierung und Anpassung der Parameter

Im Folgenden wird davon ausgegangen, dass die Elektrode 2 in der zu entkoppelnden Neuronenpopulation liegen. Der Fall, dass die Elektrode außerhalb der zu entkoppelnden Neuronenpopulation liegt wird gesondert am Ende des Abschnitts betrachtet. Für folgende Parameter der erfindungsgemäßen Vorrichtung kann beispielsweise eine Kalibrierung und Anpassung durchgeführt werden: die Frequenz der Stimulationssignale, deren Kehrwert der Stimulationsperiode entspricht, die Zeitverzögerungen der Stimulationssignale und die Intensität des Stimulationsreizes.

### 7.1 Stimulationsparameter zu Beginn der Stimulation

### 7.1.1 Frequenz, Stimulationsperiode

Wahl der Frequenz ohne vorherigen Betrieb der Vorrichtung: Der Frequenzbereich der pathologischen neuronalen Aktivität ist für die jeweiligen Krankheitsbilder dem Fachmann bekannt (Elble R.J. und Koller W.C. (1990): Tremor, John Hopkins University Press, Baltimore). Von diesem Frequenzbereich kann vorzugsweise der Mittelwert genommen werden. Alternativ kann statt dessen aus einer Datenbank der alters- und geschlechtsspezifisch zu erwartende Wert der Frequenz verwendet werden.

Es ist für den erfolgreichen Betrieb der erfindungsgemäßen Vorrichtung nicht notwendig, dass die anfänglich vorgegebene Frequenz mit der tatsächlich vorhandenen Frequenz der Aktivität der zu entkoppelnden Neuronenpopulation bzw. der Aktivität der treibenden Neuronenpopulation übereinstimmt. Die unter 7.2.1 beschriebene Regelung der Stimulationsperiode T funktioniert auch, wenn ein vom richtigen Frequenzwert stark abweichender Anfangswert verwendet wird. Hierbei bedeutet stark abweichend, dass der Wert auch um mindestens einen Faktor 10 zu groß bzw. zu klein sein kann. Alternativ kann somit auch vorzugsweise mit einem Frequenzwert begonnen werden, der in dem, dem Fachmann bekannten, für die Krankheit typischen Frequenzbereich liegt. Der Wert der Frequenz zu Beginn der Stimulation kann auch vorzugsweise durch eine individuelle Anpassung an den jeweiligen Patienten gewonnen werden. Dies kann beispielsweise durch eine die Stimulation vorbereitende Messung der neuronalen Aktivität und Abschätzung der dominierenden Frequenz der Aktivität der zu entkoppelnden und/oder der treibenden Neuronenpopulation, wie in Abschnitt 6.3 beschrieben, realisiert werden.

Wahl der Frequenz mit vorherigem Betrieb der Vorrichtung: Als Startwert für die Frequenz wird der Mittelwert der Frequenz während des vorhergehenden Betriebs der Vorrichtung gewählt.

In beiden Fällen, das heißt mit und ohne vorherigen Betrieb der Vorrichtung, wird die Stimulationsperiode T berechnet als Kehrwert des Startwerts der Frequenz.

### 7.1.2 Zeitverzögerungen

Die Zeitverzögerungen der Stimulationssignale werden vorzugsweise nach einer ersten Festlegung der Stimulationsfrequenz bzw. der Stimulationsperiode T bestimmt. Vorzugsweise werden die Zeitverzögerungen als Bruchteile der Stimulationsperiode T, z.B. T/2, gewählt. Vorzugsweise können auch Zeitverzögerungen gewählt werden, die einem Vielfachen von Bruchteilen der Stimulationsperiode T entsprechen und die Stimulationsperiode T ggf. überschreiten. Die in Abschnitt 7.2.2 beschriebene Anpassung der Zeitverzögerungen funktioniert auch in dem oben beschriebenen Fall, in dem zumindest einige der Zeitverzögerungen der Feedback-Stimulationssignale, aus denen die Stimulationsreize erzeugt werden, unterschiedlich sind und/oder die Stimulationsperiode T überschreiten.

### 7.1.3 Intensität

Die Ausgangswerte der Stimulationsparameter, welche die Intensität des Stimulationsreizes bestimmen (z. B. Verstärkung des Feedback-Stimulationssignals) werden gemäß den dem Fachmann bekannten Erfahrungswerten (z. B. maximale Amplitude 10 V) festgelegt. Die unter 7.2.3 beschriebene Regelung der Intensität funktioniert auch, wenn ein vom günstigsten Intensitätswert stark abweichender Anfangswert verwendet wird. Hierbei bedeutet stark abweichend, dass der Wert auch um mindestens einen Faktor 10 zu groß (vorzugsweise maximale Amplitude 10 V) bzw. zu klein sein kann. Alternativ kann somit auch vorzugsweise mit einem Intensitätswert begonnen werden, der in dem, dem Fachmann bekannten Bereich liegt. Insbesondere ist es bevorzugt, eine Stimulation mit kleinen Werten der Intensität, beispielsweise Maximalamplitude von 0.5 V, des Stimulationssignals zu beginnen, um somit die Nebenwirkungen der Stimulation möglicherweise zu reduzieren. Falls eine Notwenigkeit, ein stärkeres Stimulationssignal zu benutzen, besteht, kann eine Vergrößerung der Intensität in kleinen Schritten, wie in Abschnitt 7.2.3 beschrieben, durchgeführt werden.

Somit können die Anfangswerte für Frequenz und Intensität vorgegeben werden und müssen, insbesondere nicht im Rahmen einer zeitaufwendigen Kalibrierung, bestimmt werden.

### 7.2 Anpassung der Stimulationsparameter

### 7.2.1 Anpassung der Stimulationsperiode T

Im zu entkoppelnden und/oder im treibenden Areal und/oder einem damit funktionell eng verbundenen Areal wird die neuronale Aktivität gemessen, welche nach einer Bearbeitung als Stimulationssignal verwendet wird. Zum Beispiel kann beim Morbus Parkinson neben einer Messung über die Sensoren 3 direkt im zu entkoppelnden und/oder im treibenden Areal auch eine Messung der Aktivität in einem nachgeschalteten Areal, z. B. dem prämotorischen Kortex über epikortikale Sensoren erfolgen. In einem Zeitfenster mit unten angegebener Länge wird die dominante mittlere Periode bestimmt. Hierzu können unterschiedliche Algorithmen verwendet werden. Beispielsweise kann die Stimulationsperiode T an die momentane Periode der zu entkoppelnden und/oder treibenden Neuronenpopulation angepasst werden. Beispielsweise kann die momentane Periode als die zeitliche Differenz zweier nachfolgender Maxima der gemessenen neuronalen Aktivität bestimmt werden. Es kann auch beispielsweise zuerst die mittlere Frequenz der neuronalen Aktivität abgeschätzt werden, und die Stimulationsperiode *T* als Kehrwert der mittleren Frequenz festgelegt werden. Falls über Sensor 3 nicht nur krankheitsspezifische Aktivität gemessen wird, muss für diese Art der Frequenzschätzung zuerst die krankheitsspezifische Aktivität über eine Bandpassfilterung des für die Krankheit spezifischen Frequenzbereichs extrahiert werden. Alternativ kann beispielsweise die Frequenz über die in Abschnitt 6.3 genannten Frequenzschätzer bestimmt werden. Das für diese Frequenzschätzung verwendete Zeitfenster hat eine Länge, die nach oberen Werten offen sein kann und beispielsweise 10000 Perioden, vorzugsweise 1000 Perioden besonders bevorzugt 100 Perioden der krankhaften Aktivität aber auch anderen beliebigen Werten entspricht.

### 7.2.2 Anpassung der Zeitverzögerungen

Wie in den Abschnitten 3.4 und 7.1.2 beschrieben, werden die Zeitverzögerungen der Stimulationssignale vorzugsweise als Bruchteile der Stimulationsperiode T gewählt. Während der Stimulation können die Zeitverzögerungen beispielsweise fixiert sein, oder vorzugsweise an die gemäß Abschnitt 7.2.1 angepasste Stimulationsperiode angepasst werden. Um eine optimale Entkopplung und/oder Desynchronisation mit geringem resultierendem Stimulationseinfluss erreichen zu können, werden die Zeitverzögerungen der Stimulationssignale vorzugsweise während der Stimulation durch einen deterministischen oder stochastischen und/oder kombiniert stochastisch/deterministischen Algorithmus variiert. Hierzu umfasst die erfindungsgemäße Vorrichtung Mittel in Form der Steuereinheit 4, die es erlauben, die Zeitverzögerungen der Stimulationssignale während der Stimulation zu variieren.

Des Weiteren können die Zeitverzögerungen beispielsweise nicht nur innerhalb einer Stimulationsperiode sondern auch im Rahmen mehrerer Perioden variiert werden. In diesem Fall entspricht das Stimulationssignal der neuronalen Aktivität, welche zu einem einige Perioden früheren Zeitpunkt gemessen wurde.

### 7.2.3 Anpassung der Intensität

Über Sensor 3 wird die neuronale Aktivität gemessen, welches die Aktivität der zu entkoppelnden und/oder der treibenden Neuronenpopulation darstellt. Diese neuronale Aktivität wird an die Einheit 4 zur Signalverarbeitung und/oder Regelung weitergeleitet. Die Einheit 4 zur Signalverarbeitung und/oder Regelung führt eine gemäß Abschnitt 6 permanente oder wiederkehrende oder bedarfsgesteuerte Stimulation durch, wobei die Stärke des zum jeweiligen Zeitpunkt applizierten Stimulationsreizes von der Ausprägung des pathologischen Merkmals in der neuronalen Aktivität abhängt. Zu diesem Zweck kann die Intensität des Stimulationsreizes vorzugsweise angepasst werden. Bei dieser Ausführungsform umfasst die Vorrichtung eine Steuerung, die so programmiert ist, dass sie zur Regelung der Reizstärke die Verstärkung der Messsignale gemäß Abschnitt 3.3 variiert. Die relation zwischen Reizstärke und Ausprägung des pathologischen Merkmals kann entweder manuell oder in Abhängigkeit vom Stimulationserfolg automatisch geregelt werden. In einem Zeitfenster frei wählbarer, vorzugsweise konstanter Länge, das in einem konstanten Zeitabstand vor dem jeweiligen Reiz endet, wird die Ausprägung des pathologischen Merkmals in folgender Weise ermittelt:

a) In dem Fall, wenn über Sensor 3 ausschließlich bzw. überwiegend die zu entkoppelnde und/oder die treibende nathologische Aktivität gemessen wird, entspricht die Amplitude der Ausprägung der Synchronisation der zu entkoppelnden Neuronenpopulation. Die Amplitude repräsentiert somit das pathologische Merkmal. Die Amplitude kann dabei abgeschätzt werden über die Bestimmung des Maximums des Signals oder über den Mittelwert des Betrags des Signals oder mit Bandpassfilterung und/oder mit Hilbert-Transformation und/oder Wavelet-Analyse. Die ersten beiden Varianten (Bestimmung des Maximums des Signals oder Bestimmung des Mittelwerts des Betrags des Signals) werden besonders bevorzugt verwendet, da die Berechnung der Amplitude mittels Hilbert-Transformation und/oder Wavalet-Analyse einen deutlich höheren Rechenaufwand bedeutet und deren Genauigkeit von der richtigen Auswahl algorithmischer Parameter abhängt.

b) Falls über Sensor 3 neben der krankheitsspezifischen Aktivität zusätzlich noch nicht krankheitsspezifische Aktivität, zum Beispiel aus anderen Neuronenpopulationen, gemessen wird, kann für die Abschätzung der Ausprägung des pathologischen Merkmals die neuronale Aktivität nicht direkt angewandt werden. Da die krankheitsspezifische Aktivität typischerweise in einem Frequenzbereich auftritt, der von dem Frequenzbereich der nicht krankheitsspezifischen Aktivität verschieden ist, wird in diesem Fall vorzugsweise eine Abschätzung der Aktivität im krankheitsspezifischen Frequenzbereich durchgeführt. Dies wird beispielsweise durch eine Frequenzanalyse realisiert. Es kann beispielsweise die spektrale Energie im krankheitsspezifischen Frequenzbereich bestimmt werden. Alternativ hierzu kann nach Bandpassfilterung die Amplitude durch die Bestimmung des Maximums des bandpassgefilterten Signals oder durch die Bestimmung des Mittelwerts des Betrags des Signals und/oder mit Hilbert-Transformation und/oder mit Wavelet-Analyse bestimmt werden.

Wird der gewünschter Effekt nicht erzielt, das heißt, wird die zu entkoppelnde Population nicht in ausreichendem Maße entkoppelt und somit das pathologische Merkmal der neuronalen Aktivität nicht unter den Schwellenwert verschoben, wird die maximale Stärke des Stimulus bis zu einem aus Sicherheitsgründen starr vorgegebenen Maximalwert, zum Beispiel 5V, langsam erhöht (z. B. in Schritten von 0,5 V pro 50 Perioden). Hierzu verfügt die erfindungsgemäße Vorrichtung über eine Steuerung, welche eine Änderung der neuronalen Aktivität erkennt und beim Ausbleiben der Änderung der neuronalen Aktivität die stimulierenden Signale nach oberen Werten anpasst. Nach ca. 20 erfolgreichen Perioden der Stimulation kann die Vorrichtung beginnen, die maximale Stärke des Stimulus langsam (z. B. in Schritten von 0,5 V pro 50 Perioden) so lange nach unten regeln, solange der Stimulationserfolg noch vorhanden ist. Dabei wird der Stimulationserfolg wie oben beschrieben ermittelt. Die Steuerung ist dabei so programmiert, dass sie die Änderung der neuronalen Aktivität und somit den Stimulationserfolgt erkennt. Vorzugsweise wird die maximale Reizstärke auf einer Zeitskala zwischen 10 und 1000 Perioden der neuronalen Aktivität so geregelt, dass die zu entkoppelnde Neuronenpopulation ausreichend entkoppelt und desynchronisiert wird. Unabhängig von dem Wert der oben definierten Stimulationsintensität wird die Amplitude des resultierenden Stimulationseinflusses auf die zu entkoppelnde Neuronenpopulation infolge der in Abschnitt 5 beschriebenen Eigenschaften des Stimulationsmechanismus der erfindungsgemäßen Vorrichtung nach erfolgreicher Entkopplung selbständig minimiert.

7.3 Stimulationsparameter für den Fall, dass die Elektrode 2 nicht in der zu entkoppelnden Neuronenpopulation liegt

Wie im beschriebenen Fall einer nicht in der zu entkoppelnden Neuronenpopulation gelegenen Elektrode 2, wird die zu entkoppelnde Neuronenpopulation über eine indirekte Stimulation beeinflusst, wie in Abschnitt 4.1 beschrieben. Da im Fall einer indirekten Stimulation die Leitungszeiten zwischen der stimulierten Zielpopulation einerseits und der zu entkoppelnden Population andererseits jeweils verschieden groß sein können, werden vor der Durchführung der entkoppelnden Stimulation zuerst die jeweiligen Leitungszeiten gemessen. Hierzu wird über die Stimulationselektrode 2 gereizt und die Reizantwort über die, in der zu entkoppelnden Neuronenpopulation platzierten Sensoren 3, gemessen. Dies wird L mal durchgeführt, wobei L typischerweise eine kleine ganze Zahl bis zu beispielsweise 200 ist. Hieraus wird die mittlere Leitungszeit vorzugsweise in folgender Weise abgeschätzt:

Die Dauer zwischen Beginn der Stimulusapplikation über die Elektrode 2 und dem ersten Maximum der Reizantwort bzw. des Betrags der Reizantwort, $\tau^{(k)}$, wird für jede einzelne Reizapplikation bestimmt. Bei $\tau^{(k)}$ steht der Index k für den k-ten applizierten Stimulus. Hieraus wird dann für die Stimulationselektrode 2, über die indirekt stimuliert wird, separat die mittlere Dauer zwischen Reizbeginn und Reizantwort nach folgender Formel 1 bestimmt:

$$\bar{\tau} = \frac{1}{L} \sum_{k=1}^{L} \tau^{(k)} \ .$$

Formel 1

Hierbei ist L die Anzahl der über die Stimulationselektrode 2 applizierten Reize.

Für die Stimulation wird die auf diese Weise bestimmte Leitungszeit $\bar{\tau}$ in folgender Weise berücksichtigt:

Würde bei direkter Stimulation der zu entkoppelnden Neuronenpopulation mit einer Zeitverzögerung t über die Stimulationselektrode 2 ein Reiz appliziert, so wird bei indirekter Stimulation über die Stimulationselektrode 2 der Reiz mit einer Zeitverzögerung $t-\bar{\tau}$ verabreicht, wobei t größer als $\bar{\tau}$ sein muss, was gemäß Abschnitt 7.2.2 realisiert werden kann.

Die Bestimmung der Stimulationsparameter zu Beginn der Stimulation und die Regelmechanismen während der Stimulation werden unter der oben beschriebenen Berücksichtigung der Leitungszeiten $\bar{\tau}$ völlig analog, wie in den Abschnitten 7.1 und 7.2 beschrieben, durchgeführt.

## 8 Beispiele und weitere Ausführungsformen der Vorrichtung

### 8.1 Beispiele

Beispielsweise kann über die Elektrode folgender Reiz abgegeben werden:

1. Über die Elektrode wird ein Stimulationsreiz appliziert, welcher aus zwei Komponenten besteht: dem Feedback-Stimulationssignal, d.h. die bearbeitete neuronale Aktivität wobei das Stimulationssignal zeitlich um T/2 versetzt ist, wobei T die mittlere Periode der Oszillationen der zu entkoppelnden Neuronenpopulation ist. Zu diesem Signal wird die nicht zeitverzögerte bearbeitete neuronale Aktivität addiert. Gemeinsam bilden sie den Stimulationsreiz, siehe Figur 2.

2. Über die Elektrode wird ein Signal appliziert, welches aus drei Komponenten besteht: die bearbeitete und nicht zeitverzögerte neuronale Aktivität wird quadriert und mit einer um T/2 zeitverzögerten und bearbeiteten neuronalen Aktivität, wobei T die Periode des Rhythmus der treibenden Neuronenpopulation ist, multipliziert, siehe Figur 3.

Die Wirkung der Stimulation auf die zu entkoppelnde Population zeigt sich in einer Verringerung der Amplitude der gemessenen neuronalen Aktivität, siehe Figur 2a und 3a, wobei sich das Feuermuster der Neuronen, siehe Figur 2b und 3b, deutlich von dem Feuermuster im pathologischen Zustand unterscheidet. Dieser Stimulationseinfluss spiegelt sich auch im Synchronisationsmaß der zu entkoppelnden Neuronenpopulation, siehe Figur 2c und 3c, wieder, was eine Bestätigung der eintretenden Desynchronisation der zu entkoppelnden Population darstellt. Hierbei wird aufgrund der in Abschnitt 5 beschriebenen selbstregulierenden Bedarfssteuerung des Stimulationssignals die Amplitude des resultierenden Stimulationseinflusses, d.h. der Summe aus Kopplung und Stimulation, selbsttätig reduziert und minimiert, siehe Figur 2d und 3d. Des Weiteren tritt keine Beeinflussung der Eigendynamik der Neuronen während der Stimulation auf, was die Verteilung der Eigenfrequenzen der Neuronenpopulation in Figur 2e und 3e bestätigt. Als Eigenfrequenzen werden die Frequenzen der Neuronen im Zustand ohne Interaktion und ohne Stimulation verstanden. Dies bestätigt, das eine optimale Entkopplung und Desynchronisation der zu entkoppelnden Neuronenpopulation aufgrund der erfindungsgemäßen Stimulation eingetreten ist und somit die Population zu ihrem normalen funktionalen Zustand zurückgekehrt ist, was eine erhebliche Reduktion der krankheitsbedingten Symptome erwarten lässt.

Es wird beispielhaft mit drei unterschiedlichen, in Abschnitt 6 beschriebenen, Kontrollmechanismen der Reizapplikation stimuliert, mit denen vorzugsweise, wie in Abschnitt 7 beschrieben, eine bedarfsgesteuerte und somit energiesparende und milde (Nebenwirkungen vermeidende) Stimulation ermöglicht wird:

1. Permanente Reizapplikation: Es wird permanent stimuliert vorzugsweise mit Anpassung der Stimulationsperiode. Es tritt direkt nach Applikation der Stimulation eine Entkopplung und Desynchronisation der zu entkoppelnden Neuronenpopulation auf. Dadurch wird die Amplitude der gemessenen neuronalen Aktivität minimiert. Gleichzeitig tritt eine Minimierung der Amplitude des resultierenden Stimulationseinflusses auf die zu entkoppelnde Population aufgrund des in Abschnitt 5 beschriebenen Mechanismus der selbstregulierenden Bedarfssteuerung ein. Nach Ausschalten der Stimulation kann aufgrund der pathologischen Interaktion zwischen den Populationen nach kurzer Zeit eine Resynchronisation eintreten.

2. Wiederkehrende Reizapplikation, vorzugsweise mit bedarfsgesteuerter Reizstärke: Es erfolgt eine wiederkehrende Stimulation. Dabei wird die Stärke der Reize an die Stärke der Synchronisation der Neuronenpopulation angepasst: Je stärker die Kopplung und/oder Synchronisation, desto stärker ist der koordinierte Reiz.

Bei dieser Variante kann man als Zeitverzögerung statt $T/2$ vorzugsweise $\tau/2$ wählen, wobei T die Periode des Rhythmus ohne Stimulation und $\tau$ die durch Stimulation dem Rhythmus aufgezwungene Periode ist. Mit anderen Worten: $1/\tau$ ist die Frequenz des Stimulationssignals, mit der die Einzelreize appliziert werden. Hierdurch zwingt man dem System den einzigen kritischen Stimulationsparameter auf: Anstatt diesen im Rahmen einer aufwendigen Kalibrierung geeignet zu bestimmen, wird er durch die Stimulation diktiert. Außerdem wird bei dieser Form der bedarfsgesteuerten Stimulation der Umstand ausgenützt, dass die Neuronen in den betroffenen Gebieten eine (krankhafte) Tendenz zu periodischem Feuern bzw. Bursten (rhythmische Produktion von Gruppen von Aktionspotentialen) haben. Deswegen lässt sich ein Entrainment der neuronalen Aktivität der zu entkoppelnden Neuronenpopulation bezüglich der aufgezwungenen Frequenz erzielen.

3. Bedarfsgesteuerte Reizapplikation (d.h. bedarfsgesteuerte Wahl der Start- und Endzeitpunkts der Stimulation) des Stimulationsreizes: Wenn die Synchronisation der Nervenzellpopulation einen Schwellenwert überschreitet, wird der nächste Reiz über die Elektrode abgegeben, wie in Abschnitt 6.3 beschrieben.

Bei allen drei, oben beispielhaft beschriebenen, Kontrollmethoden bedingt eine in Abschnitt 5 beschriebene selbstregulierende Bedarfssteuerung eine Minimierung des Energieeintrags in die zu entkoppelnde Population. Dabei kann man vorzugsweise die einzig wichtigen Stimulationsparameter, die Stimulationsperiode T und damit die Zeitverzögerungen durch Messung der Frequenz der Nervenzellpopulation in der zu entkoppelnden und/oder der treibenden Neuronenpopulation oder einer anderen, damit eng verbundenen Nervenzellpopulation anpassen.

Es besteht die Möglichkeit, mehrere Stimulationselektroden in einer zu implantierenden Stimulationselektrode zu kombinieren, z.B. indem die Stimulationskontakte auf verschiedenen Abständen vom Ende der Elektrode positioniert sind. Dadurch kann realisiert werden, dass das zu entkoppelnde Areal möglichst umfassend stimuliert wird.

### 8.2 **Weitere Ausführungsformen der Vorrichtung**

Die erfindungsgemäße Vorrichtung kann auch zur Desynchronisation einer pathologisch synchronen Neuronenpopulation verwendet werden. Bei dieser Ausführungsform der erfindungsgemäßen Vorrichtung wird eine pathologisch synchrone Neuronenpopulation, z.B. die treibende, zu desynchronisierende Neuronenpopulation, mittels Stimulation mit dem Feedback-Stimulationssignal erfindungsgemäß desynchronisiert. Die oben beschriebene Eigenschaften der Vorrichtung und die Stimulationsmethoden für die Entkopplung der zu entkoppelnden Neuronenpopulation gelten auch für diese Ausführungsform der Vorrichtung mit der Modifikation, dass die Stimulation in der zu desynchronisierenden Neuronenpopulation appliziert wird.

Wird eine Desynchronisation angestrebt, so kann dies mit einer Anordnung der Stimulationselektrode 2 entsprechend dem Abschnitt 4.1 erzielt werden. Ebenfalls ist eine direkte und indirekte Anordnung der Sensoren 3 möglich. Die Sensoren 3 sind in diesem Fall so anzuordnen, dass eine Detektion der neuronalen Aktivität des zu desynchronisierenden Areals möglich ist. Die Details dieser Anordnung entsprechen den im Abschnitt 4.2 geschilderten Details, wobei nun die zu desynchronisierende Aktivität gemessen wird. Die pathologisch synchrone Aktivität der zu desynchronisierenden Neuronenpopulation wird gemäß Abschnitt 3 direkt und/oder indirekt gemessen und bearbeitet. Dadurch wird ein Stimulationssignal generiert, das als Grundlage für die Stimulationsreize dient. Mittels einer Stimulationselektrode werden die generierten Stimulationsreize dem zu desynchronisierenden Areal appliziert, so dass eine direkte oder indirekte Stimulation der zu desynchronisierenden Population gemäß Abschnitt 3 und 4.1 erfolgt, und die treibende Population wird erfindungsgemäß desynchronisiert, und die pathologischen Symptome werden unterdrückt. Außerdem wird, falls vorhanden, die treibende Kopplung an die getriebene Neuronenpopulation aufgrund der Desynchronisation der treibenden Population automatisch entkoppelt, d.h. durch die Desynchronisation der treibenden Neuronenpopulation entfällt der krankhafte Antrieb der getriebenen Neuronenpopulation. Aufgrund des Zusammenhangs des Stimulationssignals mit der neuronalen Aktivität der zu desynchronisierenden Neuronenpopulation wird, wie in Abschnitt 5 beschrieben, die Amplitude des resultierenden Stimulationseinflusses auf die zu desynchronisierende Population, d.h. im diesen Fall die Amplitude des Stimulationssignals (siehe Abschnitt 2), selbständig minimiert.

Die dem Ziel der Stimulation angepasste Anordnung der Elektrode und Sensoren gemäß Abschnitt 4, alle drei Kontrollmethoden der Steuerung der Reizapplikation gemäß Abschnitt 6, und die Kalibrierung und Anpassung der Parameter gemäß Abschnitt 7 können auch für die beschriebene Ausführungsform der erfindungsgemäßen Vorrichtung verwendet werden.

Die Vorrichtung kann weiterhin auch zur Entkopplung einer Neuronenpopulation verwendet werden, die durch eine nichtsynchrone pathologische Neuronenpopulation getrieben ist. Des Weiteren kann die Vorrichtung zur Entkopplung einer Population verwendet werden, die von einer pathologischen Aktivität getrieben wird und im entkoppelten Fall selbst eine nicht-pathologische synchrone Aktivität aufweist. Die Anordnung der Elektrode und Sensoren sind in diesem Fall identisch mit den in Abschnitt 4 beschriebenen Anordnungen. Auch die Detektion und Bearbeitung der neuronalen Aktivität erfolgt gemäß Abschnitt 3.

Weiterhin kann die Vorrichtung verwendet werden, um eine Kopplung eines nicht-pathologischen Areals auszuschalten, bzw. zu unterdrücken. Dies könnte z.B. in der Untersuchung der Interaktion von Neuronenpopulationen verwendet werden. Die Detektion und Bearbeitung der neuronalen Aktivität und die Anordnung der Elektrode und Sensoren geschieht hierbei entsprechend der Abschnitte 3 und 4.

Ist, wie in der Einleitung erwähnt, eine beidseitige Stimulation zwecks Entkopplung der pathologischen Aktivität notwendig, so wird vorzugsweise beidseitig mit zwei einzelnen Vorrichtungen bzw. mit einer dafür ausgelegten erfindungsgemäßen Vorrichtung stimuliert, die Signale an mindestens zwei Stimulationselektroden weitergeben kann.

## 9 Vorteile

Die erfindungsgemäße Vorrichtung hat mehrere Vorteile im Vergleich zu existierenden Vorrichtungen, z.B. DE 103 18 071.0-33 "Vorrichtung zur Desynchronisation von neuronaler Hirnaktivität":

1. Der Hauptvorteil der erfindungsgemäßen Vorrichtung besteht darin, dass ein physiologischer Reiz, nämlich das Feedback-Stimulationssignal, das heißt, die gemessene und bearbeitete neuronale Aktivität der zu entkoppelnden und/oder zu desynchronisierenden Neuronenpopulation, für die Stimulation benutzt wird. Dadurch findet die in Abschnitt 5 beschriebene selbstregulierende Bedarfssteuerung der Stimulationssignale statt, was den Energieeintrag in die zu entkoppelnde oder in die zu desynchronisierende Neuronenpopulation minimiert und zu geringen Nebenwirkungen führt.

2. Aufgrund der selbstregulierten Stimulationssignale gemäß Abschnitt 5 ist der Betrieb der erfindungsgemäßen Vorrichtung energiesparend, da sowohl, aufgrund der bedarfsgesteuerten Stimulationssignale, ein energiesparendes Signal zur Stimulation verwendet wird, als auch eine Energieeinsparung bei den für die Stimulationssteuerung notwendigen erfindungsgemäßen Steuervorrichtungen erwartet werden kann. Dadurch können längere Intervalle zwischen den notwendigen und für den Patienten beschwerlichen Batteriewechseln realisiert werden.

3. Besonders vorteilhaft ist die Ausführungsform der wiederkehrenden oder permanenten Applikation mit bedarfsgesteuerter Reizstärke, da bei diesem Verfahren keine Schwelle detektiert werden muss. Dadurch ist diese Ausführungsform mit deutlich einfacheren Algorithmen realisierbar. Dementsprechend ist ihre software- bzw. hardwaremäßige Realisation deutlich weniger aufwändig.

4. Bei permanenter und wiederkehrender Stimulation mit bedarfsgesteuerter Reizstärke und direkter Stimulation der zu entkoppelnden oder der zu desynchronisierenden Neuronenpopulation ist keine Kalibrierung nötig, d.h. es muss keine Serie von Testreizen durchgeführt werden, bei der Stimulationsparameter systematisch variiert werden, was zu einer verringerten Dauer der Kalibrierung führt.

5. Insgesamt von großem Vorteil ist die allgemeine Toleranz und Robustheit der erfindungsgemäßen Vorrichtung gegenüber der Abschätzung der Parameter wie Intensität, Stimulationsperiode und Zeitverzögerungen.

6. Durch die Verwendung von nur einer Elektrode wird der operative Aufwand und damit das Risiko einer Komplikation während der Operation für den Patienten erheblich verringert. Dadurch stellt die erfindungsgemäße Vorrichtung eine erheblich schonendere Stimulusapplikation zur Verfügung.

7. Da das zu entkoppelnde Areal vorzugsweise nahe der Hirnoberfläche lokalisiert ist, z.B. im Motorkortex, ergibt sich ein wesentlich leichterer und risikoärmerer Zugang zu den zu stimulierenden Arealen, z. B. ohne Tiefimplantation der Stimulationselektrode.

8. Die Vorrichtung kann auch zur Entkopplung nicht pathologischer Aktivität verwendet werden und bietet somit eine neuartige und wichtige Möglichkeit zur Untersuchung der Interaktion neuronaler Verbände im Gehirn.

Das Fehlen zeitaufwendiger Kalibrierung und die Stabilität der Wirkung auch bei stärkeren Frequenzschwankungen - insbesondere bei Methode 1 der Steuerung der Reizapplikation (permanente Stimulation, siehe Abschnitt 6.1) - hat wichtige Konsequenzen:

1. Schon intraoperativ lässt sich beim Plazieren Elektrode der Stimulationserfolg sofort überprüfen. Hierdurch kann das Auffinden des geeigneten Zielpunkts deutlich verbessert werden. Für die bisherigen bedarfsgesteuerten Verfahren benötigt man eine Kalibrierung, welche pro Elektrode länger als 30 Minuten dauert. Das ist intraoperativ nicht durchführbar und dem (nicht narkotisierten) Patienten nicht zumutbar.

2. Die neuen Stimulationsmethoden lassen sich auch bei neurologischen bzw. psychiatrischen Erkrankungen anwenden, bei denen pathologische Rhythmen stark schwankende Frequenzen aufweisen. Insbesondere lassen sich mit den neuen Methoden auch intermittent (d. h. kurzzeitig auftretende) Rhythmen entkoppeln. Hieraus ergibt sich, dass die neuen Stimulationsmethoden bei weit mehr Erkrankungen zur Anwendung kommen können, vor allem auch bei Epilepsien.

Mit der erfindungsgemäßen Vorrichtung können mit dem neuen Stimulationsverfahren folgende Krankheiten bzw. Symptome durch Entkopplung geeigneter Hirnareale behandelt werden.

Bei allen neurologischen und psychiatrischen Erkrankungen, bei denen pathologische neuronale Synchronisation eine für die Ausprägung der krankheitsspezifischen Symptome relevante Rolle spielt, zum Beispiel: Morbus Parkinson, essentieller Tremor, Dystonie, Zwangserkrankungen, Tremor bei Multipler Sklerose, Tremor in Folge eines Schlaganfalls oder einer anderen, zum Beispiel tumorösen Gewebsschädigung, zum Beispiel im Bereich des Thalamus und/oder der Basalganglien, Choreoathetose und Epilepsie, wobei die Aufzählung nicht einschränkend sein soll.

Bei der zur Zeit verwendeten Standardmethode, der Hochfrequenz-Dauerstimulation, werden folgende Zielareale beispielhaft verwendet:

Bei Morbus Parkinson der Nucleus subthalamicus oder bei tremordominantem Morbus Parkinson der Thalamus, zum Beispiel der Nucleus ventralis intermedius thalami.

Bei essentiellem Tremor der Thalamus, zum Beispiel der Nucleus ventralis intermedius thalami.

Bei Dystonie und Choreoathetose das Globus pallidum internum bei Epilepsie der Nucleus subthalamicus, das Kleinhirn, thalamische Kerngebiete, zum Beispiel der Nucleus ventralis intermedius thalami, oder der Nucleus caudatus.

Bei Zwangserkrankungen die Capsula interna oder der Nucleus accumbens.

Bei der erfindungsgemäßen Vorrichtung können beispielsweise die für die jeweiligen Erkrankungen oben aufgeführten Zielareale und/oder damit gekoppelte Areale gewählt werden. Weil bei der erfindungsgemäßen Vorrichtung entweder keine Kalibrierung notwendig ist oder die Kalibrierung sehr schnell durchgeführt werden kann, ergibt sich die Möglichkeit, im Rahmen der Elektrodenimplantation alternative Zielareale auszutesten, bei denen sich die entkoppelnde und/oder die desynchronisierende Wirkung der erfindungsgemäßen Vorrichtung noch besser entfalten lässt.

Die Erfindung umfasst ebenfalls eine Steuerung, welche die angegebene Funktionsweise der erfindungsgemäßen Vorrichtung steuert sowie die Verwendung der Vorrichtung und der Steuerung für die Behandlung der Krankheiten Morbus Parkinson, essentieller Tremor, Dystonie, Zwangserkrankungen, Choreoathetose, Tremor bei Multipler Sklerose, Tremor in Folge eines Schlaganfalls oder einer anderen, zum Beispiel tumorösen Gewebeschädigung, zum Beispiel im Bereich des Thalamus und/oder der Basalganglien, und Epilepsie.

Die erfindungsgemäße Vorrichtung kann sowohl als Implantat zur dauerhaften Therapie der obengenannten neurologischen und psychiatrischen Erkrankungen als auch für die intraoperative Zielpunkt-Diagnostik, das heißt, die intraoperative Auffindung des optimalen Zielpunkts für die Elektrodenimplantation, verwendet werden.

**Patentansprüche**

1. Vorrichtung zur Entkopplung und/oder Desynchronisation von neuronaler Hirnaktivität, mit

   - mindestens einem Sensor (3) zur Messung mindestens eines Signals, welches die zeitliche Entwicklung der Aktivität der zu entkoppelnden und/oder der zu desynchronisierenden Neuronenpopulation wiedergibt, sowie
   - genau einer Elektrode (2),

   **gekennzeichnet durch**

   - eine Steuerung (4), welche derart ausgestaltet ist, dass sie die Messsignale des Sensors (3) aufnimmt und die Messsignale als Stimulationssignale und Stimulationsreize in die Elektrode (2) einspeist oder in einer alternativen Ausgestaltung die Messsignale bearbeitet und anschließend als Stimulationssignale und Stimulationsreize in die Elektrode (2) einspeist.

2. Vorrichtung nach Anspruch 1, **dadurch gekennzeichnet,**

   - **dass** die Steuerung (4) über die Sensoren (3) den zeitlichen Verlauf der Aktivität der zu entkoppelnden und/oder zu desynchronisierenden Neuronenpopulation direkt und/oder indirekt misst, wobei
   - die Steuerung (4) insbesondere über mindestens einen der Sensoren (3) den zeitlichen Verlauf der Aktivität einer durch das zu entkoppelnde und/oder zu desynchronisierende Areal beeinflussten Muskelgruppe und/oder den zeitlichen Verlauf der Aktivität einer dem zu entkoppelnden und/oder zu desynchronisierenden Areal zugeordneten Neuronenpopulation misst.

3. Vorrichtung nach Anspruch 2, **dadurch gekennzeichnet,**

   - **dass** die Steuerung (4) den zeitlichen Verlauf der Aktivität permanent oder in einer alternativen Ausgestaltung in zeitlich begrenzten Messintervallen misst, wobei die Steuerung (4) bei einer Messung in zeitlich begrenzten Messintervallen insbesondere die Dauer und/oder die Abstände der begrenzten Messintervalle durch einen deterministischen und/oder stochastischen und/oder kombiniert stochastisch/deterministischen Algorithmus steuert, und/oder
   - **dass** die Steuerung (4) die Messsignale speichert.

4. Vorrichtung nach einem der Ansprüche 1 bis 3, **dadurch gekennzeichnet,**

   - **dass** die Steuerung (4) die Messsignale bearbeitet, indem sie Stimulationssignale erzeugt, die gegenüber den Messsignalen zeitverzögert sind, wobei die Steuerung (4) insbesondere Stimulationssignale erzeugt, deren Zeitverzögerungen Bruchteilen oder Vielfachen der Bruchteile der Periode der Messsignale entsprechen und/oder wobei die Steuerung (4) insbesondere Stimulationssignale mit wenigsten teilweise unterschiedlichen Zeitverzögerungen erzeugt, und/oder
   - **dass** die Steuerung (4) die Messsignale bearbeitet, indem sie die Messsignale filtert, und/oder
   - **dass** die Steuerung (4) die Messsignale bearbeitet, indem sie für die Messsignale eine Frequenzanalyse und/oder eine Bandpassfilterung und/oder eine Wavelet-Analyse und/oder eine Hilbert-Transformation und/oder Filterung in zeitlicher Domäne durchführt, und/oder
   - **dass** die Steuerung (4) die Messsignale bearbeitet, indem sie die Messsignale linear und/oder nichtlinear kombiniert und/oder transformiert, wobei die Steuerung (4) insbesondere die Messsignale untereinander und/oder mit sich selbst multipliziert, dividiert, addiert und/oder subtrahiert und/oder mittels anderer nichtlinearer Funktionen transformiert und/oder den Betrag bildet, und/oder
   - **dass** die Steuerung (4) die Messsignale bearbeitet, indem sie die Messsignale verstärkt, und/oder
   - **dass** die Steuerung (4) die Messsignale bearbeitet, indem sie die Polarität der Messsignale verändert, und/oder
   - **dass** die Steuerung (4) die Messsignale bearbeitet, indem sie die Messsignale zeitlich kodiert, wobei die Steuerung (4) insbesondere die Messsignale als Pulszüge, insbesondere als Niedrig- oder Hochfrequenzpulszüge, kodiert, und/oder
   - **dass** die Steuerung (4) die Messsignale bearbeitet, indem sie Stimulationssignale erzeugt, die mit gleichen Bearbeitungsschritten generiert werden, oder dass die Steuerung (4) in einer alternativen Ausgestaltung mindestens zwei Stimulationssignale mit unterschiedlichen Bearbeitungsschritten erzeugt, und/oder
   - **dass** die Steuerung (4) die Bearbeitungsschritte und/oder deren Parameter zeitlich durch einen deterministischen und/oder stochastischen und/oder kombiniert stochastisch/deterministischen Algorithmus ändert, und/oder
   - **dass** die Steuerung (4) die maximale Amplitude des Stimulationssignals beschränkt.

**5.** Vorrichtung nach einem der Ansprüche 1 bis 4, **dadurch gekennzeichnet,**

- **dass** die Steuerung (4) die Elektrode (2) mit Stimulationsreizen ansteuert, wobei die Steuerung (4) insbesondere aus den Stimulationssignalen Stimulationsreize erzeugt.

**6.** Vorrichtung nach Anspruch 5, **dadurch gekennzeichnet,**

- **dass** die Steuerung (4) die Stimulationssignale untereinander und/oder mit sich selbst multipliziert, dividiert, addiert und/oder subtrahiert und/oder mittels anderer nichtlinearer Funktionen transformiert und/oder Betrag bildet um Stimulationsreize zu generieren, und/oder
- **dass** die Steuerung (4) Stimulationsreize erzeugt, deren Nettoladungseintrag im wesentlichen Null ist, und/oder
- **dass** die Steuerung (4) die Applikation der Stimulationsreize zeitlich steuert, wobei die Steuerung (4) insbesondere die Stimulationsreize permanent oder in einer alternativen Ausgestaltung wiederkehrend appliziert und wobei die Steuerung (4) bei einer wiederkehrenden Applikation insbesondere die Stimulationsreize in zeitlich begrenzten Stimulationsintervallen appliziert und dabei die Steuerung (4) insbesondere die Dauer und/oder die Abstände der Stimulationsintervalle durch einen deterministischen und/oder stochastischen und/oder kombiniert stochastisch/deterministischen Algorithmus steuert.

**7.** Vorrichtung nach einem der Ansprüche 1 bis 6, **dadurch gekennzeichnet,**

- **dass** die Steuerung (4) eine zusätzliche Bedarfssteuerung beinhaltet.

**8.** Vorrichtung nach Anspruch 7, **dadurch gekennzeichnet,**

- **dass** die Steuerung (4) die Messsignale und/oder die Stimulationssignale zur Bedarfssteuerung verwendet, und/oder
- **dass** die Steuerung (4) ein pathologisches Merkmal in dem Mess- und/oder Stimulationssignal detektiert, und/oder
- **dass** die Steuerung (4) die Amplitude des Mess- und/oder Stimulationssignals zur Bedarfssteuerung verwendet, und/oder
- **dass** die Steuerung (4) die Amplitude des Mess- und/oder Stimulationssignals abschätzt, indem sie das Signal selbst und/oder den Betrag des Signals und/oder das im krankheitsspezifischen Frequenzbereich bandpassgefilterte Signal und/oder den Betrag des im krankheitsspezifischen Frequenzbereich bandpassgefilterten Signals und/oder die mit Hilbert-Transformation und/oder Wavelet-Analyse bestimmte instantane Amplitude verwendet, und/oder
- **dass** die Steuerung (4) bei Detektion eines pathologischen Merkmals in dem Mess- und/oder Stimulationssignal einen Stimulationsreiz appliziert, und/oder
- **dass** die Steuerung (4) ein pathologisches Merkmal dadurch detektiert, dass sie das Überschreiten eines Schwellenwerts der Amplitude des Mess- und/oder Stimulationssignals detektiert, und/oder
- **dass** die Steuerung (4) ein pathologisches Merkmal dadurch detektiert, dass sie das Überschreiten eines Schwellenwerts der Amplitude des im krankheitsspezifischen Frequenzbereich bandpassgefilterten Messsignals und/oder des Stimulationssignals detektiert, und/oder
- **dass** die Steuerung (4) zur Detektion eines pathologischen Merkmals die Amplitude des Mess- und/oder Stimulationssignals mit dem Schwellenwert in einem gleitenden Zeitfenster vergleicht.

**9.** Vorrichtung nach einem der Ansprüche 5 bis 8, **dadurch gekennzeichnet,**

- **dass** die Steuerung (4) die Elektrode (2) mit gleichen Stimulationsreizen ansteuert oder in einer alternativen Ausgestaltung mit unterschiedlichen Stimulationsreizen ansteuert, wobei die Steuerung (4) bei einer Ansteuerung mit unterschiedlichen Stimulationsreizen insbesondere unterschiedliche Stimulationsreize erzeugt, die aus unterschiedlichen Stimulationssignalen und/oder mittels unterschiedlicher Transformationen und/oder Kombinationen der Stimulationssignale generiert werden, und/oder wobei die Steuerung (4) bei einer Ansteuerung mit unterschiedlichen Stimulationsreizen insbesondere die Reihenfolge und/oder die Art und/oder den Energieeintrag und/oder die Parameter der Stimulationsreize mit einem deterministischen und/oder stochastischen und/oder kombiniert stochastisch/deterministischen Algorithmus ermittelt und variiert.

**10.** Vorrichtung nach einem der Ansprüche 1 bis 9, **dadurch gekennzeichnet,**

- **dass** die Steuerung (4) die Parameter der Stimulationssignale und/oder Stimulationsreize verändert.

**11.** Vorrichtung nach Anspruch 10, **dadurch gekennzeichnet,**

- **dass** die Steuerung (4) die Parameter der Stimulationssignale und/oder Stimulationsreize verändert, indem die Stimulationsperiode T an die momentane Periode der zu entkoppelnden und/oder zu desynchronisierenden Neuronenpopulation anpasst, wobei die Steuerung (4) insbesondere die momentane Periode entweder über eine Abschätzung der zeitlichen Differenz von Triggerpunkten oder mittels Frequenzschätzern bestimmt, und/oder

- **dass** die Steuerung (4) die Parameter der Stimulationssignale und/oder Stimulationsreize verändert, indem sie die Stimulationsperiode T an die mittlere Frequenz der zu entkoppelnden und/oder zu desynchronisierenden Neuronenpopulation anpasst, und/oder

- **dass** die Steuerung (4) die Parameter der Stimulationssignale und/oder Stimulationsreize verändert, indem sie die Zeitverzögerung der Stimulationssignale an die Stimulationsperiode T anpasst, und/oder

- **dass** die Steuerung (4) die Parameter der Stimulationssignale und/oder Stimulationsreize verändert, indem sie die Reizstärke anpasst, wobei die Steuerung (4) insbesondere die Reizstärke auf einer Zeitskala zwischen 10 und 1000 Perioden der neuronalen Aktivität so regelt, dass eine ausreichende Entkopplung und/oder Desynchronisation auftritt, und/oder wobei die Steuerung (4) insbesondere zur Regelung der Reizstärke die Verstärkung der Messsignale variiert, und/oder

- **dass** die Steuerung (4) so programmiert ist, dass die Relation zwischen Reizstärke und Ausprägung des pathologischen Merkmals entweder manuell einstellbar ist und/oder in Abhängigkeit vom Stimulationserfolg automatisch geregelt wird.

12. Vorrichtung nach einem der Ansprüche 1 bis 11, **dadurch gekennzeichnet,**

- **dass** die Steuerung (4) eine zusätzliche manuelle Bedarfssteuerung beinhaltet, und/oder
- **dass** die Steuerung (4) die Mess- und Stimulationsintervalle überlappend oder zeitgleich oder zeitlich getrennt anordnet, und/oder
- **dass** die Steuerung (4) die zu entkoppelnde und/oder zu desynchronisierende Neuronenpopulation entweder direkt oder indirekt über die Elektrode (2) stimuliert, wobei die Steuerung (4) insbesondere eine mit der zu entkoppelnden und/oder zu desynchronisierenden Neuronenpopulation über Nervenfaserbündel verbundene Neuronenpopulation stimuliert wird, und/oder ein mit der zu entkoppelnden und/oder zu desynchronisierenden Neuronenpopulation verbundenes Nervenfaserbündel über die Elektrode (2) stimuliert, und/oder

- **dass** die Steuerung (4) Unterschiede in der Leitungszeit zwischen dem Reizort der Elektrode (2) und dem Ort der von ihr stimulierten Neuronenpopulation detektiert, und/oder

- **dass** die Steuerung (4) bei der Berechnung der Zeitverzögerungen der Stimulationssignale und/oder bei der Bearbeitung der Messsignale die zugehörigen Leitungszeiten mit verrechnet, und/oder

- **dass** die Elektrode (2) mit mindestens einem Sensor (3) baulich vereint ist, und/oder

- **dass** eine galvanische Trennung zwischen der Steuerung (4) und den Elektroden (2) besteht, und/oder

- **dass** die Vorrichtung mit Mitteln zur Visualisierung und Verarbeitung der Mess- und/oder Stimulationssignale sowie zur Datensicherung über den Telemetriesender (11) und den Telemetrieempfänger (12) in Verbindung steht, und/oder

- **dass** die Vorrichtung über einen Telemetriesender (11) und Telemetrieempfänger (13) mit einer zusätzlichen Referenzdatenbank in Verbindung steht.

**Claims**

1. An apparatus for decoupling and/or desynchronizing neural brain activity, comprising:

- at least one sensor (3) for measuring at least one signal which reproduces the development in time of the activity of the neuronal population to be decoupled and/or to be desynchronized; and
- exactly one electrode (2),

**characterized by**

- a control (4) which is configured such that it receives the measurement signals of the sensor (3) and feeds the measurement signals into the electrode (2) as stimulation signals and stimulation stimuli; or, in an alternative embodiment, processes the measurement signals and subsequently feeds them into the electrode (2) as stimulation signals and stimulation stimuli.

2. An apparatus in accordance with claim 1, **characterized in that**

- the control (4) measures time development of the activity of the neuronal population to be decoupled and/or to be desynchronized directly and/or indirectly via the sensors (3), wherein
- the control (4) in particular measures via at least one of the sensors (3) the time development of the activity of a muscle group influenced by the area to be decoupled and/or to be desynchronized and/or the time development of the activity of a neuronal population associated with the area to be decoupled and/or to be desynchronized.

**3.** An apparatus in accordance with claim 2, **characterized in that** the control (4) measures the time development of the activity permanently; or, in an alternative embodiment, measures in time-limited measurement intervals, with the control (4), on a measurement in time-limited measuring intervals, in particular controlling the duration and/or the intervals of the limited measurement intervals by means of a deterministic and/or stochastic algorithm and/or a combined stochastic/deterministic algorithm; and/or

- **in that** the control (4) stores the measurement signals.

**4.** An apparatus in accordance with any one of the claims 1 to 3, **characterized in that**

- the control (4) processes the measurement signals by generating stimulation signals which are time delayed with respect to the measurement signals, with the control (4) in particular generating stimulation signals whose time delays correspond to fractions or multiples of the fractions of the period of the measurement signals, and/or with the control (4) in particular generating stimulation signals with at least partially different time delays; and/or
- **in that** the control (4) processes the measurement signals by filtering the measurement signals; and/or
- **in that** the control (4) processes the measurement signals by carrying out a frequency analysis and/or a band-pass filtering and/or a wavelet analysis and/or Hilbert transformation and/or by filtering in the time domain for the measurement signals; and/or
- **in that** the control (4) processes the measurement signals by combining and/or transforming the measurement signals linearly and/or nonlinearly, with the control (4) in particular multiplying, dividing, adding and/or subtracting the measurement signals by, with and/or from one another and/or by, with and/or from themselves and/or transforming them by means of other nonlinear functions and/or forming the sum; and/or
- **in that** the control (4) processes the measurement signals by amplifying the measurement signals; and/or

- **in that** the control (4) processes the measurement signals by changing the polarity of the measurement signals; and/or
- **in that** the control (4) processes the measurement signals by time-coding the measurement signals, with the control (4) in particular coding the measurement signals as pulse trains, in particular as low or high frequency pulse trains; and/or
- **in that** the control (4) processes the measurement signals by generating stimulation signals which are generated with the same processing steps, or **in that**, in an alternative embodiment, the control (4) generates at least two stimulation signals with different processing steps; and/or
- **in that** the control (4) changes the processing steps and/or their parameters in time by means of a deterministic and/or stochastic and/or combined stochastic/deterministic algorithm; and/or
- **in that** the control (4) limits the maximum amplitude of the stimulation signal.

**5.** An apparatus in accordance with any one of the claims 1 to 4, **characterized in that**

- the control (4) controls the electrode (2) with stimulation stimuli, with the control (4) in particular generating stimulation stimuli from the stimulation signals.

**6.** An apparatus in accordance with claim 5, **characterized in that**

- the control (4) multiplies, divides, adds and/or subtracts the stimulation signals by, with and/or from one another and/or by, with and/or from themselves and/or transforms them by means of other nonlinear functions and/or forms the sum, in order to generate stimulation stimuli; and/or
- **in that** the control (4) generates stimulation stimuli, the net charge input of which is essentially zero; and/or
- **in that** the control (4) controls the application of the stimulation stimuli in time, with the control (4) applying the stimulation stimuli permanently, or, in an alternative embodiment, repetitively, and with the control (4), in a repetitive application, in particular applying the stimulation stimuli in stimulation intervals limited in time and in this respect the control (4) in particular controlling the duration and/or the intervals of the stimulation intervals by means of a deterministic and/or stochastic algorithm and/or a combined stochastic/deterministic algorithm.

**7.** An apparatus in accordance with any one of the claims 1 to 6,

**characterized in that**
the control (4) includes an additional demand control.

8. An apparatus in accordance with claim 7, **characterized in that**

- the control (4) uses the measurement signals and/or the stimulation signals for demand control; and/or
- **in that** the control (4) detects a pathological feature in the measurement signal and/or stimulation signal; and/or
- **in that** the control (4) uses the amplitude of the measurement signal and/or stimulation signal for the demand control; and/or
- **in that** the control (4) estimates the amplitude of the measurement signal and/or stimulation signal by using the signal itself and/or the sum of the signal and/or the signal which is band-pass-filtered in the disease-specific frequency range and/or the sum of the signal which is band-pass-filtered in the disease-specific frequency range and/or the instantaneous amplitude determined by Hilbert transformation and/or by wavelet analysis; and/or
- **in that** the control (4), on detecting a pathological feature in the measurement signal and/or stimulation signal, applies a stimulation stimulus; and/or
- **in that** the control (4) detects a pathological feature by detecting the exceeding of a threshold value of the amplitude of the measurement signal and/or stimulation signal; and/or
- **in that** the control (4) detects a pathological feature by detecting the exceeding of a threshold value of the amplitude of the measurement signal, which is band-pass-filtered in the disease-specific frequency range, and/or of the stimulation signal; and/or
- **in that** the control (4), for detecting a pathological feature, compares the amplitude of the measurement signal and/or stimulation signal with the threshold value in a sliding time window.

9. An apparatus in accordance with any one of the claims 5 to 8, **characterized in that**

- the control (4) controls the electrode (2) with the same stimulation stimuli, or, in an alternative embodiment, with different stimulation stimuli, with the control (4), in the case of an activation with different stimulation stimuli, in particular generating different stimuli which are generated from different stimulation signals and/or by means of different transformations and/or combinations of the stimulation signals, and/or with the control (4), in the case of an activation with different stimulation stimuli, determining and varying the order and/or the type and/or the energy input and/or the parameters of the stimulation stimuli by means of a deterministic and/or stochastic algorithm and/or a combined stochastic/deterministic algorithm.

10. An apparatus in accordance with any one of the claims 1 to 9, **characterized in that**

- the control (4) changes the parameters of the stimulation signals and/or stimulation stimuli.

11. An apparatus in accordance with claim 10, **characterized in that**

- the control (4) changes the parameters of the stimulation signals and/or stimulation stimuli by adapting the stimulation period T to the instantaneous period of the neuronal population to be decoupled and/or to be desynchronized, with the control (4) in particular determining the instantaneous period either by estimating the time difference of trigger points or by means of frequency estimators; and/or
- **in that** the control (4) changes the parameters of the stimulation signals and/or stimulation stimuli by adapting the stimulation period T to the mean frequency of the neuronal population to be decoupled and/or to be desynchronized; and/or
- **in that** the control (4) changes the parameters of the stimulation signals and/or stimulation stimuli by adapting the time delay of the stimulation signals to the stimulation period $T$; and/or
- **in that** the control (4) changes the parameters of the stimulation signals and/or stimulation stimuli by adapting the stimulus intensity, with the control (4) in particular regulating the stimulus intensity on a time scale between 10 and 1000 periods of the neural activity in such a manner that adequate decoupling and/or desynchronization occurs, and/or with the control (4) in particular varying the amplification of the measurement signals for controlling the stimulus intensity; and/or
- **in that** the control (4) is programmed in such a manner that the relation between stimulus intensity and instance of the pathological feature can either be adjusted manually and/or is controlled automatically in dependence on the stimulation result.

12. An apparatus in accordance with any one of the claims 1 to 11, **characterized in that**

- the control (4) includes an additional manual demand control; and/or
- **in that** the control (4) arranges the measurement intervals and stimulation intervals as overlapping or at the same time or separately in time; and/or

- **in that** the control (4) stimulates the neuronal population to be decoupled and/or to be desynchronized either directly or indirectly via the electrode (2), with the control (4) in particular stimulating a neuronal population connected via nerve fiber bundles with the neuronal population to be decoupled and/or to be desynchronized and/or stimulating a nerve fiber bundle, connected to the neuronal population to be decoupled and/or to be desynchronized, via the electrode (2); and/or

- **in that** the control (4) detects differences in the conduction time between the stimulation location of the electrode (2) and the location of the neuronal population stimulated by it; and/or

- **in that** the control (4), on calculating the time delays of the stimulation signals and/or on processing the measurement signals, calculates in the associated conduction times; and/or

- **in that** the electrode (2) is constructionally combined with at least one sensor (3); and/or

- **in that** a galvanic isolation exists between the control (4) and the electrodes (2); and/or

- **in that** the apparatus is connected to means for displaying and processing the measurement signals and/or stimulation signals and for saving data via the telemetry transmitter (11) and the telemetry receiver (12); and/or

- **in that** the apparatus is connected to an additional reference database via a telemetry transmitter (11) and a telemetry receiver (13).

**Revendications**

1. Dispositif pour découpler et/ou désynchroniser l'activité cérébrale neuronale, comportant

- au moins un capteur (3) pour mesurer au moins un signal qui reflète l'évolution temporelle de l'activité de la population neuronale à découpler et/ou à désynchroniser, ainsi que
- exactement une électrode (2),

**caractérisé par**

- une commande (4) qui est réalisée de manière à recevoir les signaux de mesure du capteur (3) et à injecter dans l'électrode (2) les signaux de mesure en tant que signaux de stimulation et de stimuli ou, selon une variante de réalisation, à traiter les signaux de mesure et ensuite à les injecter dans l'électrode (2) en tant que signaux de stimulation et de stimuli.

2. Dispositif selon la revendication 1, **caractérisé en ce que**

- au moyen des capteurs (3), la commande (4) mesure directement et/ou indirectement l'évolution temporelle de l'activité de la population neuronale à découpler et/ou à désynchroniser,
- la commande (4) mesure, en particulier par l'un des capteurs (3), l'évolution temporelle de l'activité d'un groupe musculaire influencé par l'aire à découpler et/ou à désynchroniser, et/ou l'évolution temporelle de l'activité d'une population neuronale associée à l'aire à découpler et/ou à désynchroniser.

3. Dispositif selon la revendication 2, **caractérisé en ce que**

- la commande (4) mesure l'évolution temporelle de l'activité en permanence ou, selon une variante de réalisation, à des intervalles de mesure limités dans le temps, et
- lors d'une mesure à des intervalles de mesure limités dans le temps, la commande (4) contrôle en particulier la durée et/ou les écarts des intervalles de mesure limités par un algorithme déterministe et/ou stochastique et/ou combiné stochastique/déterministe, et/ou
- la commande (4) met en mémoire les signaux de mesure.

4. Dispositif selon l'une des revendications 1 à 3, **caractérisé en ce que**

- la commande (4) traite les signaux de mesure en générant des signaux de stimulation qui sont retardés dans le temps par rapport aux signaux de mesure, la commande (4) générant en particulier des signaux de stimulation dont les retards correspondent à des fractions ou à des multiples des fractions de la période des signaux de mesure, et/ou la commande (4) générant en particulier des signaux de stimulation avec des retards au moins partiellement différents, et/ou
- **en ce que** la commande (4) traite les signaux de mesure en filtrant les signaux de mesure, et/ou
- **en ce que** la commande (4) traite les signaux de mesure en effectuant pour les signaux de mesure une analyse de fréquence et/ou un filtrage passe-bande et/ou une analyse des ondelettes et/ou une transformée de Hilbert et/ou un filtrage dans le domaine temporel, et/ou
- **en ce que** la commande (4) traite les signaux de mesure en combinant et/ou en transformant les signaux de mesure de façon linéaire et/ou non-linéaire, et la commande (4) multiplie, divise, additionne et/ou soustrait les signaux de mesure entre eux et/ou par eux-mêmes, et/ou les transforme au moyen d'autres fonctions non-linéaires et/ou forme la valeur absolue et/ou

- **en ce que** la commande (4) traite les signaux de mesure en amplifiant les signaux de mesure, et/ou

- **en ce que** la commande (4) traite les signaux de mesure en modifiant la polarité des signaux de mesure, et/ou

- **en ce que** la commande (4) traite les signaux de mesure en effectuant un codage temporel des signaux de mesure, la commande (4) effectuant un codage des signaux de mesure en particulier en tant que trains d'impulsions, notamment en tant que trains d'impulsions à basse ou à haute fréquence, et/ou

- **en ce que** la commande (4) traite les signaux de mesure en générant des signaux de stimulation qui sont générés dans des étapes de traitement identiques, ou **en ce que** la commande (4) génère, selon une variante de réalisation, au moins deux signaux de stimulation par des étapes de traitement différentes, et/ou

- **en ce que** la commande (4) change temporellement les étapes de traitement et/ou leurs paramètres par un algorithme déterministe et/ou stochastique et/ou combiné stochastique/déterministe, et/ou

- **en ce que** la commande (4) restreint l'amplitude maximale du signal de stimulation.

5. Dispositif selon l'une des revendications 1 à 4, **caractérisé en ce que**

- la commande (4) pilote l'électrode (2) par des stimuli, la commande (4) générant des stimuli en particulier à partir des signaux de stimulation.

6. Dispositif selon la revendication 5, **caractérisé en ce que**

- la commande (4) multiplie, divise, additionne et/ou soustrait les signaux de stimulation entre eux et/ou par eux-mêmes, et/ou les transforme au moyen d'autres fonctions non-linéaires et/ou forme la valeur absolue, pour générer des stimuli, et/ou

- **en ce que** la commande (4) génère des stimuli dont l'apport énergétique net est sensiblement nul, et/ou

- **en ce que** la commande (4) contrôle temporellement l'application des stimuli, et la commande (4) applique les stimuli en particulier en permanence ou, selon une variante de réalisation, de façon récurrente, et lors d'une application récurrente la commande (4) applique les stimuli en particulier à des intervalles de stimulation limités dans le temps, et la commande (4) contrôle en particulier la durée et/ou les écarts des intervalles de stimulation par un algorithme déterministe et/ou stochastique et/ou combiné stochastique/déterministe.

7. Dispositif selon l'une des revendications 1 à 6, **caractérisé en ce que**

- la commande (4) inclut une commande supplémentaire en fonction des besoins.

8. Dispositif selon la revendication 7, **caractérisé en ce que**

- la commande (4) utilise les signaux de mesure et/ou les signaux de stimulation pour la commande en fonction des besoins, et/ou

- **en ce que** la commande (4) détecte une caractéristique pathologique dans le signal de mesure et/ou de stimulation, et/ou

- **en ce que** la commande (4) utilise l'amplitude du signal de mesure et/ou de stimulation pour la commande en fonction des besoins, et/ou

- **en ce que** la commande (4) estime l'amplitude du signal de mesure et/ou de stimulation du fait qu'elle utilise le signal lui-même et/ou la valeur absolue du signal et/ou le signal filtré en passe-bande dans la plage de fréquence spécifique à la maladie et/ou la valeur absolue du signal filtré en passe-bande dans la plage de fréquence spécifique à la maladie et/ou l'amplitude instantanée déterminée par la transformée de Hilbert et/ou par l'analyse des ondelettes, et/ou

- **en ce que** lors de la détection d'une caractéristique pathologique dans le signal de mesure et/ou de stimulation, la commande (4) applique un stimulus, et/ou

- **en ce que** la commande (4) détecte une caractéristique pathologique en détectant le dépassement d'une valeur seuil de l'amplitude du signal de mesure et/ou de stimulation, et/ou

- **en ce que** la commande (4) détecte une caractéristique pathologique en détectant le dépassement d'une valeur seuil de l'amplitude du signal de mesure et/ou du signal de stimulation filtré en passe-bande dans la plage de fréquence spécifique à la maladie, et/ou

- **en ce que** pour la détection d'une caractéristique pathologique la commande (4) compare l'amplitude du signal de mesure et/ou de stimulation à la valeur seuil dans une fenêtre temporelle glissante.

9. Dispositif selon l'une des revendications 5 à 8, **caractérisé en ce que**

- la commande (4) pilote l'électrode (2) par des stimuli identiques ou, selon une variante de réalisation, par des stimuli différents, et lors d'un pilotage par des stimuli différents, la commande (4) génère en particulier des stimuli différents qui sont générés à partir de signaux de stimulation différents et/ou au moyen de transformées et/ou de combinaisons différentes de signaux de stimulation, et/ou lors d'un pilotage par des stimuli différents la commande (4) détermine et varie en particulier l'ordre et/ou le type et/ou l'apport d'énergie et/ou les paramètres des stimuli par un algorithme déterministe et/ou stochastique et/ou combiné stochastique/déterministe.

**10.** Dispositif selon l'une des revendications 1 à 9, **caractérisé en ce que**

- la commande (4) modifie les paramètres des signaux de stimulation et/ou des stimuli.

**11.** Dispositif selon la revendication 10, **caractérisé en ce que**

- la commande (4) modifie les paramètres des signaux de stimulation et/ou des stimuli en adaptant la période de stimulation T à la période momentanée de la population neuronale à découpler et/ou à désynchroniser, et la commande (4) détermine en particulier la période momentanée soit par estimation de la différence temporelle de points de déclenchement soit par des organes d'estimation de fréquence, et/ou
- **en ce que** la commande (4) modifie les paramètres des signaux de stimulation et/ou des stimuli en adaptant la période de stimulation T à la fréquence moyenne de la population neuronale à découpler et/ou à désynchroniser, et/ou
- **en ce que** la commande (4) modifie les paramètres des signaux de stimulation et/ou des stimuli en adaptant le retard temporel des signaux de stimulation à la période de stimulation T, et/ou
- **en ce que** la commande (4) modifie les paramètres des signaux de stimulation et/ou des stimuli en les adaptant à l'intensité des stimuli, la commande (4) régulant en particulier l'intensité des stimuli sur une échelle de temps entre 10 et 1000 périodes de l'activité neuronale, de telle sorte qu'il résulte un découplage et/ou une désynchronisation suffisant(e), et/ou la commande (4) faisant varier l'amplification des signaux de mesure en particulier pour réguler l'intensité des stimuli, et/ou
- **en ce que** la commande (4) est programmée de telle sorte que la relation entre l'intensité des stimuli et l'ampleur de la caractéristique pathologique est réglable manuellement et/ou est réglée automatiquement en fonction du succès de stimulation.

**12.** Dispositif selon l'une des revendications 1 à 11, **caractérisé en ce que**

- la commande (4) inclut une commande supplémentaire manuelle en fonction des besoins, et/ou
- **en ce que** la commande (4) agence les intervalles de mesure et de stimulation soit en chevauchement, soit simultanément, soit encore de façon séparée dans le temps, et/ou
- **en ce que** la commande (4) stimule la population neuronale à découpler et/ou à désynchroniser soit directement soit indirectement par l'électrode (2), la commande (4) stimulant en particulier une population neuronale reliée par des faisceaux de fibres nerveuses à la population neuronale à découpler et/ou à désynchroniser et/ou stimule par l'électrode (2) un faisceau de fibres nerveuses relié à la population neuronale à découpler et/ou à désynchroniser, et/ou
- **en ce que** la commande (4) détecte des différences dans la durée de conduction entre l'endroit de stimulation de l'électrode (2) et l'endroit de la population neuronale stimulée par celle-ci, et/ou
- **en ce que** lors du calcul des retards temporels des signaux de stimulation et/ou lors du traitement des signaux de mesure, la commande (4) prend en compte les durées de conduction associées, et/ou
- **en ce que** l'électrode (2) est structurellement réunie avec au moins un capteur (3), et/ou
- **en ce qu'**il existe une séparation galvanique entre la commande (4) et les électrodes (2), et/ou
- **en ce que** le dispositif est en communication avec des moyens destinés à la visualisation et au traitement des signaux de mesure et/ou de stimulation ainsi qu'à la sauvegarde via l'émetteur de télémétrie (11) et le récepteur de télémétrie (12), et/ou
- **en ce que** le dispositif est en communication avec une base supplémentaire de données de référence via un émetteur de télémétrie (11) et un récepteur de télémétrie (13).

Fig. 1

Fig. 2

Fig. 3

treibende,

zu desynchronisierende

Neuronenpopulation

getriebene,

zu entkoppelnde

Neuronenpopulation

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10211766 A1
- DE 10318071033

**In der Beschreibung aufgeführte Nicht-Patentliteratur**

- **ELBLE R.J. ; KOLLER W.C.** Tremor. John Hopkins University Press, 1990